# EUROPEAN PATENT APPLICATION

(11) **EP 1 331 275 A1**
(43) Date of publication of application: **30.07.2003**
(21) Application number: 01976869.6
(22) Date of filing: 30.10.2001
(51) Int. Cl.: C12Q 1/68

(54) **METHOD OF DETERMINING NUCLEIC ACID BASE SEQUENCE**

(30) Priority: 30.10.2000 JP 2000331513
(71) Applicant: TAKARA BIO INC., Otsu-shi, Shiga 520-2193 (JP)
(72) Inventor: UEMORI, Takashi, Otsu-shi, Shiga 520-2141 (JP); YAMASHITA, Hiroshige, Yokkaichi-shi, Mie 510-0829 (JP); HOKAZONO, Shigekazu 205, Takara, Otsu-shi, Shiga 520-2134 (JP); SATO, Yoshimi, Ritto-shi, Shiga 520-3031 (JP); MUKAI, Hiroyuki, Moriyama-shi, Shiga 524-0102 (JP); ASADA, Kiyozo, Koka-gun, Shiga 520-3333 (JP); KATO, Ikunoshin, Uji-shi, Kyoto 611-0028 (JP)
(74) Representative: Grund, Martin, Dr.
(86) International application number: JP0109493
(87) International publication number: WO02036822

(57) **Abstract**

A method of determining the base sequence of a nucleic acid characterized by involving: the step of amplifying a template nucleic acid in the presence of at least two primers each having a tag sequence, a primer specific to the template nucleic acid and a DNA polymerase, wherein the primers having tag sequences have each the tag sequence in the 5'-terminal side thereof and a specific base sequence consisting of three or more nucleotides in the 3'-terminal side; and the step of directly sequencing the amplified fragments obtained in the above step.

SEQUENCE LISTING

<110> Takara Bio Inc.

<120> Method of Determining Nucleic Acid Base Sequence

<130> 38-58

<150> JP 2000-331513
<151> 2000-10-30

<160> 28

<210> 1
<211> 17
<212> DNA
<213> Artificial Sequence

<220>
<223> Artificially designed oligonucleotide

<400> 1
ggcacgattc gataacg 17

<210> 2
<211> 16
<212> DNA
<213> Artificial Sequence

<220>
<223> Artificially designed oligonucleotide

<400> 2
ggcacgattc gataac                                16

<210> 3
<211> 17
<212> DNA
<213> Artificial Sequence

<220>
<223> Artificially designed oligonucleotide

<400> 3
caggaaacag ctatgac                               17

<210> 4
<211> 37
<212> DNA
<213> Artificial Sequence

<220>
<223> Artificially designed oligonucleotide

<400> 4
ggtggcgcga tgcaaatgca atcttcgttg ccccaac                               37

<210> 5

<211> 30

<212> DNA

<213> Artificial Sequence

<220>

<223> E6 sph primer

<400> 5

tggccttcga gcgatgcatg ctcactgcca 30

<210> 6

<211> 35

<212> DNA

<213> Artificial Sequence

<220>

<223> Artificially designed oligonucleotide

<400> 6

tttccaatgg agggttctag atgaacgaag gtgaa 35

<210> 7

<211> 33

<212> DNA

<213> Artificial Sequence

<220>

<223> Artificially designed oligonucleotide

<400> 7

cgacatagtg aggtgtctag acggaaagaa gga                    33

<210> 8

<211> 30

<212> DNA

<213> Artificial Sequence

<220>

<223> Artificially designed oligonucleotide

<400> 8

tttacacttt atgcttccgg ctcgtatgtt                    30

<210> 9

<211> 30

<212> DNA

<213> Artificial Sequence

<220>

<223> Artificially designed oligonucleotide

<400> 9

ccttatctat gatctccttc tttccgtctg                    30

<210> 10

<211> 23

<212> DNA

<213> Artificial Sequence

<220>

<223> PCR primer RN-F1 for cloning a gene encoding a polypeptide having

a RNaseHII activity from Thermococcus litoralis

<400> 10

ggcattgatg aggctggnar rgg    23

<210> 11

<211> 23

<212> DNA

<213> Artificial Sequence

<220>

<223> PCR primer RN-R0 for cloning a gene encoding a polypeptide having

a RNaseHII activity from Thermococcus litoralis

<400> 11

gtccttggat cgctgggrta ncc    23

<210> 12

<211> 24

<212> DNA

<213> Artificial Sequence

<220>

<223> PCR primer TliRN-1 for cloning a gene encoding a polypeptide

having a RNaseHII activity from Thermococcus litoralis

<400> 12

tagctttttt gaatctttga ctcc    24

<210> 13

<211> 24

<212> DNA

<213> Artificial Sequence

<220>

<223> PCR primer TliRN-2 for cloning a gene encoding a polypeptide

having a RNaseHII activity from Thermococcus litoralis

<400> 13

ctgctgcatc aatactagct aaag    24

<210> 14

<211> 24

<212> DNA

<213> Artificial Sequence

<220>

<223> PCR primer TceRN-1 for cloning a gene encoding a polypeptide

having a RNaseHII activity from Thermococcus celer

<400> 14

tctctgagct tcggaacgtt cttc    24

<210> 15

<211> 24

<212> DNA
<213> Artificial Sequence

<220>
<223> PCR primer TceRN-2 for cloning a gene encoding a polypeptide
having a RNaseHII activity from Thermococcus celer

<400> 15
acccgtgaca gggcgataga aaag    24

<210> 16
<211> 17
<212> DNA
<213> Artificial Sequence

<220>
<223> Designed oligonucleotid as tag sequence.

<400> 16
ggcacgattc gataacg    17

<210> 17
<211> 675
<212> DNA
<213> Thermococcus litoralis

<400> 17
atgaagctgg gaggaataga tgaagccggc aggggaccag ttataggccc tcttgtaatt    60
gcagcggttg ttgtcgatga atcccgtatg caggagcttg aagctttggg agtcaaagat   120

tcaaaaaagc taacaccaaa aagaagagaa gagctatttg aggagattgt gcaaatagtt 180

gatgaccacg ttatcattca gctttcccca gaggagatag acggcagaga tggtacaatg 240

aacgagcttg aaattgaaaa ctttgccaaa gcgttgaact cccttaaagt taagccggat 300

gtgctctaca tagatgcggc cgatgtcaag gaaaagcgct ttggcgacat tataggtgaa 360

agactttcct tctctccaaa gataatcgcc gaacataagg cagattcaaa gtacattcca 420

gtggctgctg catcaatact agctaaagtt acccgtgaca gggcaataga gaagctcaag 480

gagctttatg gggagatagg ctcaggatat ccaagtgatc caaatacaag gaggtttctg 540

gaggagtatt acaaggctca tggggaattc cccccaatag tgaggaaaag ctggaagacc 600

cttagaaaga tagaagaaaa actaaaagct aaaaagactc agcccactat cttggacttc 660

ttaaaaaagc cttaa 675

<210> 18

<211> 224

<212> PRT

<213> Thermococcus litoralis

<400> 18

Met Lys Leu Gly Gly Ile Asp Glu Ala Gly Arg Gly Pro Val Ile

1               5                   10                  15

Gly Pro Leu Val Ile Ala Ala Val Val Val Asp Glu Ser Arg Met

                20                  25                  30

Gln Glu Leu Glu Ala Leu Gly Val Lys Asp Ser Lys Lys Leu Thr

                35                  40                  45

Pro Lys Arg Arg Glu Glu Leu Phe Glu Glu Ile Val Gln Ile Val

                50                  55                  60

Asp Asp His Val Ile Ile Gln Leu Ser Pro Glu Glu Ile Asp Gly

                65                  70                  75

Arg Asp Gly Thr Met Asn Glu Leu Glu Ile Glu Asn Phe Ala Lys

                80                  85                  90

Ala Leu Asn Ser Leu Lys Val Lys Pro Asp Val Leu Tyr Ile Asp
                95                  100                 105
Ala Ala Asp Val Lys Glu Lys Arg Phe Gly Asp Ile Ile Gly Glu
                110                 115                 120
Arg Leu Ser Phe Ser Pro Lys Ile Ile Ala Glu His Lys Ala Asp
                125                 130                 135
Ser Lys Tyr Ile Pro Val Ala Ala Ala Ser Ile Leu Ala Lys Val
                140                 145                 150
Thr Arg Asp Arg Ala Ile Glu Lys Leu Lys Glu Leu Tyr Gly Glu
                155                 160                 165
Ile Gly Ser Gly Tyr Pro Ser Asp Pro Asn Thr Arg Arg Phe Leu
                170                 175                 180
Glu Glu Tyr Tyr Lys Ala His Gly Glu Phe Pro Pro Ile Val Arg
                185                 190                 195
Lys Ser Trp Lys Thr Leu Arg Lys Ile Glu Glu Lys Leu Lys Ala
                200                 205                 210
Lys Lys Thr Gln Pro Thr Ile Leu Asp Phe Leu Lys Lys Pro
                215                 220

<210> 19
<211> 39
<212> DNA
<213> Artificial Sequence

<220>
<223> PCR primer TliNde for amplifying a gene encoding a polypeptide
having a RNaseHII activity from Thermococcus litoralis

<400> 19

gaggaggtag gcatatgaag ctgggaggaa tagatgaag   39

<210> 20

<211> 39

<212> DNA

<213> Artificial Sequence

<220>

<223> PCR primer TliBam for amplifying a gene encoding a polypeptide

having a RNaseHIII activity from Thermococcus litoralis

<400> 20

aaaggaaacc ttcggatcca ttaaggcttt tttaagaag   39

<210> 21

<211> 702

<212> DNA

<213> Thermococcus celer

<400> 21

ttgaagctcg caggaataga cgaggctgga aggggccccg taatcggccc gatggtcatc   60

gcggccgtcg tcctcgatga gaagaacgtt ccgaagctca gagatctcgg cgtcagggac   120

tcgaaaaagc tgaccccaaa gaggagggag agattattta acgacataat taaacttttg   180

gatgattatg taattcttga attatggccg gaggagatag actcccgcgg cgggacgctt   240

aacgagctcg aggtggagag gttcgtggag gccctcaact cgcttaaggt gaagcccgac   300

gtcgtttaca tagacgcggc ggacgtgaag gagggccgct ttggcgagga gataaaggaa   360

aggttgaact tcgaggcgaa gattgtctca gagcacaggg cggacgataa gtttttaccg   420

gtgtcctctg cctcgatact ggcgaaggtg acccgtgaca gggcgataga aaagctcaag   480

gagaagtacg gcgagatcgg gagcggctac ccgagcgacc caaggacgag ggagttcctc   540

gagaactact acagacaaca cggcgagttc ccgcccgtag tccggcgaag ctggaagacg 600
ctgagaaaga tagaggaaaa gctgaggaaa gaggccgggt caaaaaaccc ggagaattca 660
aaggaaaagg gacagacgag cctggacgta tttttgaggt ag 702

<210> 22
<211> 233
<212> PRT
<213> Thermococcus celer

<400> 22
Leu Lys Leu Ala Gly Ile Asp Glu Ala Gly Arg Gly Pro Val Ile
1               5                   10                  15
Gly Pro Met Val Ile Ala Ala Val Val Leu Asp Glu Lys Asn Val
                20                  25                  30
Pro Lys Leu Arg Asp Leu Gly Val Arg Asp Ser Lys Lys Leu Thr
                35                  40                  45
Pro Lys Arg Arg Glu Arg Leu Phe Asn Asp Ile Ile Lys Leu Leu
                50                  55                  60
Asp Asp Tyr Val Ile Leu Glu Leu Trp Pro Glu Glu Ile Asp Ser
                65                  70                  75
Arg Gly Gly Thr Leu Asn Glu Leu Glu Val Glu Arg Phe Val Glu
                80                  85                  90
Ala Leu Asn Ser Leu Lys Val Lys Pro Asp Val Val Tyr Ile Asp
                95                  100                 105
Ala Ala Asp Val Lys Glu Gly Arg Phe Gly Glu Glu Ile Lys Glu
                110                 115                 120
Arg Leu Asn Phe Glu Ala Lys Ile Val Ser Glu His Arg Ala Asp
                125                 130                 135
Asp Lys Phe Leu Pro Val Ser Ser Ala Ser Ile Leu Ala Lys Val

                140                 145                 150

Thr Arg Asp Arg Ala Ile Glu Lys Leu Lys Glu Lys Tyr Gly Glu

                155                 160                 165

Ile Gly Ser Gly Tyr Pro Ser Asp Pro Arg Thr Arg Glu Phe Leu

                170                 175                 180

Glu Asn Tyr Tyr Arg Gln His Gly Glu Phe Pro Pro Val Val Arg

                185                 190                 195

Arg Ser Trp Lys Thr Leu Arg Lys Ile Glu Glu Lys Leu Arg Lys

                200                 205                 210

Glu Ala Gly Ser Lys Asn Pro Glu Asn Ser Lys Glu Lys Gly Gln

                215                 220                 225

Thr Ser Leu Asp Val Phe Leu Arg

                230

<210> 23

<211> 39

<212> DNA

<213> Artificial Sequence

<220>

<223> PCR primer TceNde for amplifying a gene encoding a polypeptide

having a RNaseHII activity from Thermococcus celer

<400> 23

caggggtga gcatatgaag ctcgcaggaa tagacgagg 39

<210> 24

<211> 39

<212> DNA

<213> Artificial Sequence

<220>

<223> PCR primer TceBam for amplifying a gene encoding a polypeptide

having a RNaseHIII activity from Thermococcus celer

<400> 24

tgaacccgcg taggatccta cctcaaaaat acgtccagg   39

<210> 25

<211> 675

<212> DNA

<213> Thermococcus litoralis

<400> 25

atgaagctgg gaggaataga tgaagccggc aggggaccag ttataggccc tcttgtaatt   60

gcagcggttg ttgtcgatga atcccgtatg caggagcttg aagctttggg agtcaaagat   120

tcaaaaaagc taacaccaaa aagaagagaa gagctatttg aggagattgt gcaaatagtt   180

gatgaccacg ttatcattca gctttcccca gaggagatag acggcagaga tggtacaatg   240

aacgagcttg aaattgaaaa ctttgccaaa gcgttgaact cccttaaagt taagccggat   300

gtgctctaca tagatgcggc cgatgtcaag gaaaagcgct ttggcgacat tataggtgaa   360

agactttcct tctctccaaa gataatcgcc gaacataagg cagattcaaa gtacattcca   420

gtggctgctg catcaatact agctaaagtt acccgtgaca gggcaataga gaagctcaag   480

gagttttatg gggagatagg ctcaggatat ccaagtgatc caattacaag gaggtttctg   540

gaggagtatt acaaggctca tggggaattc cccccaatag tgaggaaaag ctggaagacc   600

cttagaaaga tagaagaaaa actaaaagct aaaaagactc agcccactat cttggacttc   660

ttaaaaaagc cttaa   675

<210> 26

<211> 224

<212> PRT

<213> Thermococcus litoralis

<400> 26

Met Lys Leu Gly Gly Ile Asp Glu Ala Gly Arg Gly Pro Val Ile
1               5                   10                  15

Gly Pro Leu Val Ile Ala Ala Val Val Val Asp Glu Ser Arg Met
                20                  25                  30

Gln Glu Leu Glu Ala Leu Gly Val Lys Asp Ser Lys Lys Leu Thr
                35                  40                  45

Pro Lys Arg Arg Glu Glu Leu Phe Glu Glu Ile Val Gln Ile Val
                50                  55                  60

Asp Asp His Val Ile Ile Gln Leu Ser Pro Glu Glu Ile Asp Gly
                65                  70                  75

Arg Asp Gly Thr Met Asn Glu Leu Glu Ile Glu Asn Phe Ala Lys
                80                  85                  90

Ala Leu Asn Ser Leu Lys Val Lys Pro Asp Val Leu Tyr Ile Asp
                95                  100                 105

Ala Ala Asp Val Lys Glu Lys Arg Phe Gly Asp Ile Ile Gly Glu
                110                 115                 120

Arg Leu Ser Phe Ser Pro Lys Ile Ile Ala Glu His Lys Ala Asp
                125                 130                 135

Ser Lys Tyr Ile Pro Val Ala Ala Ala Ser Ile Leu Ala Lys Val
                140                 145                 150

Thr Arg Asp Arg Ala Ile Glu Lys Leu Lys Glu Phe Tyr Gly Glu
                155                 160                 165

Ile Gly Ser Gly Tyr Pro Ser Asp Pro Ile Thr Arg Arg Phe Leu
                170                 175                 180

Glu Glu Tyr Tyr Lys Ala His Gly Glu Phe Pro Pro Ile Val Arg
                185                 190                 195
Lys Ser Trp Lys Thr Leu Arg Lys Ile Glu Glu Lys Leu Lys Ala
                200                 205                 210
Lys Lys Thr Gln Pro Thr Ile Leu Asp Phe Leu Lys Lys Pro
                215                 220

<210> 27
<211> 702
<212> DNA
<213> Thermococcus celer

<400> 27
atgaagctcg cagaaataga cgaggctgga aggggccccg taatcggccc gatggtcatc 60
gcggccgtcg tcctcgatga gaagaacgtt ccgaagctca gagatctcgg cgtcagggac 120
tcgaaaaagc tgaccccaaa gaggagggag agattattta acgacataat taaacttttg 180
gatgattatg taattcttga attatggccg gaggagatag actcccgcgg cgggacgctt 240
aacgagctcg aggtggagag gttcgtggag gccctcaact cgcttaaggt gaagcccgac 300
gtcgtttaca tagacgcggc ggacgtgaag gagggccgct ttggcgagga gataaaggaa 360
aggttgaact tcgaggcgaa gattgtctca gagcacaggg cggacgataa gtttttaccg 420
gtgtcctctg cctcgatact ggcgaaggtg acccgtgaca gggcgataga aaagctcaag 480
gagaagtacg gcgagatcgg gagcggctac ccgagcgacc caaggacgag ggagttcctc 540
gagaactact acagacaaca cggcgagttc ccgcccgtag tccggcgaag ctggaagacg 600
ctgagaaaga tagaggaaaa gctgaggaaa gaggccgggt caaaaaaccc ggagaattca 660
aaggaaaagg gacagacgag cctggacgta tttttgaggt ag 702

<210> 28
<211> 233
<212> PRT

<213> Thermococcus celer

<400> 28

Met Lys Leu Ala Glu Ile Asp Glu Ala Gly Arg Gly Pro Val Ile
1               5                   10                  15

Gly Pro Met Val Ile Ala Ala Val Val Leu Asp Glu Lys Asn Val
                20                  25                  30

Pro Lys Leu Arg Asp Leu Gly Val Arg Asp Ser Lys Lys Leu Thr
                35                  40                  45

Pro Lys Arg Arg Glu Arg Leu Phe Asn Asp Ile Ile Lys Leu Leu
                50                  55                  60

Asp Asp Tyr Val Ile Leu Glu Leu Trp Pro Glu Glu Ile Asp Ser
                65                  70                  75

Arg Gly Gly Thr Leu Asn Glu Leu Glu Val Glu Arg Phe Val Glu
                80                  85                  90

Ala Leu Asn Ser Leu Lys Val Lys Pro Asp Val Val Tyr Ile Asp
                95                  100                 105

Ala Ala Asp Val Lys Glu Gly Arg Phe Gly Glu Glu Ile Lys Glu
                110                 115                 120

Arg Leu Asn Phe Glu Ala Lys Ile Val Ser Glu His Arg Ala Asp
                125                 130                 135

Asp Lys Phe Leu Pro Val Ser Ser Ala Ser Ile Leu Ala Lys Val
                140                 145                 150

Thr Arg Asp Arg Ala Ile Glu Lys Leu Lys Glu Lys Tyr Gly Glu
                155                 160                 165

Ile Gly Ser Gly Tyr Pro Ser Asp Pro Arg Thr Arg Glu Phe Leu
                170                 175                 180

Glu Asn Tyr Tyr Arg Gln His Gly Glu Phe Pro Pro Val Val Arg
                185                 190                 195

Arg Ser Trp Lys Thr Leu Arg Lys Ile Glu Glu Lys Leu Arg Lys
                200                 205                 210
Glu Ala Gly Ser Lys Asn Pro Glu Asn Ser Lys Glu Lys Gly Gln
                215                 220                 225
Thr Ser Leu Asp Val Phe Leu Arg
                230

## Description

### Technical Field

The present invention relates to a method for determining a nucleotide sequence of a nucleic acid which is useful in a field of genetic engineering.

### Background Art

Currently, the mainstream method for analyzing a nucleotide sequences of a nucleic acid is a chain terminator method in which the analysis is carried out by electrophoresis using plate-type or capillary-type gel. The length of a nucleotide sequence that can be analyzed at a time in the method has been increased as a result of improvements in the polymerase and the electrophoresis equipment to be used. Nevertheless, the length that can be analyzed is usually only about 500 base pairs, and at the most 1000 base pairs or less. Therefore, in order to determine a nucleotide sequence of a DNA fragment longer than several kilo base pairs which has been cloned into a conventional vector (plasmid, phage, cosmid, etc.), one needs to use one of a primer walking method, a subcloning method and a deletion clone construction method, or a combination thereof.

For example, if a nucleotide sequence of a DNA fragment of five kilo base pairs in length which has been cloned in a plasmid vector is to be determined using the primer walking method, a nucleotide sequence is determined first from one of the termini of the cloned DNA fragment using a primer having a nucleotide sequence on the vector. Another primer is then designed and synthesized based on the newly obtained nucleotide sequence information to determine a nucleotide sequence of a region beyond the region of the previously determined nucleotide sequence. The entire nucleotide sequence of the cloned fragment can be determined by repeating the above-mentioned step several times. However, since the primer walking method requires designing and synthesis of a primer at every step of nucleotide sequence determination, it requires a lot of time and cost.

If a nucleotide sequence of such a DNA fragment is to be analyzed using the subcloning method, the plasmid DNA is first digested with various restriction enzymes to prepare a restriction map for the cloned DNA fragment based on the lengths of the DNA fragments resulting from the digestions. DNA fragments obtained by digestion with restriction enzyme(s) selected based on the restriction map are subcloned into a phage or plasmid vector. Then, the nucleotide sequences are determined using a primer having a sequence on the vector. Since the subcloning method requires a complicated procedure including preparation of a restriction map and subsequent subclonings, it requires a lot of labor and time. In addition, restriction enzyme recognition sites suitable for subcloning need to be uniformly distributed on the original cloned DNA fragment in order to efficiently determine the nucleotide sequence of the DNA fragment according to this method.

In the deletion clone construction method which is a nucleotide sequence determination method developed by Yanisch-Perron et al. as described in Gene, 33:103-119 (1985), a series of clones are prepared by successively shortening the cloned fragment from one of the termini of the fragment as a basic point. According to this method, the problems associated with the primer walking method and the subcloning method are partially solved. Specifically, the deletion clone construction method does not require designing and synthesis of a primer at every step of nucleotide sequence determination which are required according to the primer walking method, or preparation of a restriction map and subcloning based on the restriction map which are required according to the subcloning method. However, the deletion clone construction method requires considerable skill in genetic engineering because sequential treatments of the plasmid having the cloned DNA fragment with two restriction enzymes, exo III nuclease, exo VII nuclease, Klenow fragment DNA polymerase and DNA ligase in this order under conditions suitable for the respective enzymes are required in order to prepare a series of clones with successively shortened DNA fragments according to this method. Furthermore, it is necessary to determine the reactivity (liability to deletion) of the cloned DNA fragment to exo III nuclease by carrying out a preliminary experiment before the final sequential treatments because the reactivity varies depending on the nucleotide sequence of the cloned DNA fragment.

Methods in which nucleotide sequences of fragments randomly amplified by a PCR are analyzed include the degenerate oligonucleotide-primed PCR (DOP-PCR) method of Telenius et al. (Genomics, 13:718-725 (1992)) and the tagged random hexamer amplification (TRHA) method of Wong et al. (Nucleic Acids Research, 24(19):3778-3783 (1996)). In each method, a PCR is carried out using a mixture of plural primers containing random sequences, and multiple amplified laddered bands are isolated and purified one by one, and then subjected to sequencing. Therefore, the procedures of these methods are complicated.

As described above, all of the current methods for analyzing a nucleotide sequence of a nucleic acid have problems. Thus, a rapid and low-cost method for analyzing a nucleotide sequence of a nucleic acid has been desired for analyses of genomes.

### Objects of Invention

The main object of the present invention is to provide a method for determining a nucleotide sequence of a nucleic acid in which a series of amplified DNA fragments whose lengths from one basic point on a template nucleic acid are successively shortened is prepared without a complicated procedure, and the nucleotide sequences of the DNA fragments are analyzed.

### Summary of Invention

As a result of intensive studies, the present inventors have found that a series of amplified DNA fragments of varying lengths from one basic point on a template DNA can be prepared by carrying out PCRs using a primer specific for a template and primers selected from a pool of primers consisting of plural primers having defined nucleotide sequences in combination. The present inventors have demonstrated that the entire nucleotide sequence of the original DNA fragment can be analyzed by determining the nucleotide sequences of the respective DNA fragments in the series of amplified DNA fragments. Thus, the present invention has been completed.

The first aspect of the present invention relates to a method for determining a nucleotide sequence of a nucleic acid, the method comprising:
(1) amplifying a template nucleic acid in the presence of each one of at least two primers each having a tag sequence, a primer specific for the template nucleic acid and a DNA polymerase, wherein the primer having a tag sequence has the tag sequence on the 5'-terminal side and a defined nucleotide sequence of three or more nucleotides on the 3'-terminal side; and
(2) subjecting a fragment amplified in step (1) above to direct sequencing.

According to the first aspect, a primer having a structure represented by General Formula can be used as the primer having a tag sequence:
General Formula: 5'-tag sequence-Sₐ-3'
wherein "S" represents one nucleotide or a mixture of two or more nucleotides selected from the group consisting of G, A, T and C, and "a" represents an integer of three or more, provided that at least three S's in "Sₐ" represent one nucleotide selected from the group consisting of G, A, T and C.

According to the first aspect, a primer selected from the primers listed in Tables 1 to 5 can be used as the primer having a tag sequence.

According to the first aspect, the amplification of the template nucleic acid is carried out, for example, using a polymerase chain reaction (PCR). A pol I-type, α-type or non-pol I, non-α-type DNA polymerase, or a mixture of DNA polymerases can be preferably used as the DNA polymerase in the PCR, and the DNA polymerase can be selected from the group consisting of Taq DNA polymerase, Pfu DNA polymerase, Ex-Taq DNA polymerase, LA-Taq DNA polymerase, Z-Taq DNA polymerase, Tth DNA polymerase, KOD DNA polymerase and KOD dash DNA polymerase.

The method of the first aspect may further comprise selecting a reaction that generates a substantially single-banded PCR-amplified fragment. The method may further comprise selecting a substantially single-banded PCR-amplified fragment.

The first aspect may be carried out directly on a sample or after preparing a template nucleic acid from a sample. A template nucleic acid in a form of a plasmid, phage, cosmid, BAC or YAC library, or a genomic DNA or cDNA may be preferably used.

The second aspect of the present invention relates to a pool of primers used for the method for determining a nucleotide sequence of a nucleic acid of the first aspect, which contains at least two primers each having a tag sequence on the 5'-terminal side and a defined nucleotide sequence of three or more nucleotides on the 3'-terminal side.

A primer having a structure represented by General Formula can be preferably used as the primer having a tag sequence in the pool of primers of the second aspect:
General Formula: 5'-tag sequence-Sₐ-3'
wherein "S" represents one nucleotide or a mixture of two or more nucleotides selected from the group consisting of G, A, T and C, and "a" represents an integer of three or more, provided that at least three S's in "Sₐ" represent one nucleotide selected from the group consisting of G, A, T and C.

According to the second aspect, the tag sequence in the primer may contain a sequence of a primer for sequencing.

The third aspect of the present invention relates to a composition for determining a nucleotide sequence of a nucleic acid, which contains the pool of primers of the second aspect.

The composition of the third aspect may further contain a DNA polymerase. A pol I-type, α-type or non-pol I, non-α-type DNA polymerase, or a mixture of DNA polymerases can be used as the DNA polymerase. For example, Taq DNA polymerase, Ex-Taq DNA polymerase, LA-Taq DNA polymerase, Z-Taq DNA polymerase, KOD DNA polymerase or KOD dash DNA polymerase can be preferably used.

The fourth aspect of the present invention relates to a kit used for the method for determining a nucleotide sequence of a nucleic acid of the first aspect, which contains the pool of primers of the second aspect.

The kit of the fourth aspect may further contain a DNA polymerase and a buffer for the DNA polymerase.

The kit of the fourth aspect may be in a packed form and contain instructions that direct use of the pool of primers of the second aspect and the above-mentioned DNA polymerase. The respective primers each having a tag sequence in the pool of primers may be dispensed in predetermined positions.

The fifth aspect of the present invention relates to a product consisting of a packing material and a reagent for determining a nucleotide sequence of a nucleic acid enclosed in the packing material, wherein the reagent contains a pool of primers and/or a DNA polymerase, and wherein description that the reagent can be used for determination of a nucleotide sequence is indicated in a label stuck to the packing material or instructions attached to the packing material.

### Detailed Description of the Invention

The present invention is described below with respect to a case where a polymerase chain reaction (PCR) is used for amplifying a template nucleic acid. However, the amplification method to be used according to the present invention is not limited to the PCR. Any method that can be used to specifically amplify a region in a template nucleic acid defined by two primers in the presence of a DNA polymerase may be used. Examples of such methods include the ICAN method (WO 00/56877), the SDA method (Japanese Patent No. 2087497) and the RCA method (United States Patent No. 5854035).

As used herein, a primer refers to an oligonucleotide containing a deoxyribonucleotide or a ribonucleotide such as an adenine nucleotide (A), a guanine nucleotide (G), a cytosine nucleotide (C) or a thymine nucleotide (T). The deoxyribonucleotides may comprise an unmodified or modified deoxyribonucleotide as long as it can be used in a PCR.

As used herein, a 3'-terminal side refers to a portion from the center to the 3' terminus of a nucleic acid such as a primer. Likewise, a 5'-terminal side refers to a portion from the center to the 5' terminus of a nucleic acid.

As used herein, a tag sequence refers to a nucleotide sequence that is common among respective primers contained in a pool of primers, or that is different from primer to primer in a pool of primers, and is positioned on the 5'-terminal side of the primer or in a portion from the center to the 3' terminus of the primer. Although it is not intended to limit the present invention, the tag sequence may contain a sequence to which a sequencing primer for a chain terminator method anneals, or a recognition site for a restriction endonuclease. It is preferable to select a nucleotide sequence that hardly anneals to a template nucleic acid for the tag sequence. However, it is not intended to limit the present invention because it may be difficult to select such a nucleotide sequence depending on the sequence of the template DNA.

Hereinafter, the present invention will be described in detail.

### (1) The pool of primers of the present invention

The pool of primers of the present invention is a library of primers each having a tag sequence at the 5' terminus and being capable of annealing to an arbitrary nucleotide sequence. A sequence on the 3'-terminal side of a primer selected from a pool of primers is mainly important for extension of a DNA strand from the primer in a PCR. In addition, it is effective for specific amplification upon a PCR to select a nucleotide sequence that hardly anneals to a template for the tag sequence. A primer contained in a pool of primers used in the method of the present invention has a nucleotide sequence that is substantially complementary to an arbitrary nucleotide sequence in a template nucleic acid, and enables extension of a DNA strand from its 3' terminus. A DNA strand may be extended even if the nucleotide sequence on the 3'-terminal side of the primer is not completely complementary to the template DNA. It is usually preferable to design primers such that the primers in a pool can anneal to portions almost uniformly distributed on a template nucleic acid having an arbitrary nucleotide sequence. As used herein, "a substantially complementary nucleotide sequence" means a nucleotide sequence capable of annealing to a DNA as a template under reaction conditions used. For example, such a primer can be designed according to "Labo Manual PCR" (published by Takara Shuzo, pp. 13-16, 1996). Alternatively, a commercially available software for designing a primer such as OLIGO™ Primer Analysis software (Takara Shuzo) may be used.

Although it is not intended to limit the present invention, the length of the oligonucleotide primer used in the method of the present invention is preferably from about 15 nucleotides to about 100 nucleotides, more preferably from about 18 nucleotides to about 40 nucleotides. The nucleotide sequence of the primer is preferably substantially complementary to a template nucleic acid such that it anneals to the template nucleic acid under reaction conditions used.

Although it is not intended to limit the present invention, for example, an oligonucleotide having a structure represented by General Formula below can be used as a primer according to the present invention:
General Formula: 5'-tag sequence-Sₐ-3'
wherein "S" represents one nucleotide or a mixture of two or more nucleotides selected from the group consisting of G, A, T and C, and "a" represents an integer of three or more, provided that at least three S's in "Sₐ" represent one nucleotide selected from the group consisting of G, A, T and C.

For example, a nucleotide sequence of preferably 10 or more nucleotides, more preferably 15 or more nucleotides is placed as a tag sequence on the 5'-terminal side of a primer. Although there is no specific limitation concerning the sequence of the tag sequence, it preferably does not form a secondary structure or a dimeric structure. A sequence that is not complementary to a nucleotide sequence of a template nucleic acid is particularly preferable. If information on a nucleotide sequence of a nucleic acid as a template is available, a tag sequence can be designed with reference to the information. For example, a tag sequence can be selected from a set of about fifty sequences each consisting of six nucleotides that are found at the lowest frequencies in a template nucleic acid. Although it is not intended to limit the present invention, for example, a specific nucleotide sequence GGCACGATTCGATAACG (SEQ ID NO:1) can be selected as a tag sequence if a nucleotide sequence from Escherichia coli, a bacterium belonging to genus Pyrococcus or a bacterium belonging to genus Bacillus is to be analyzed.

A defined nucleotide sequence on the 3'-terminal side of a primer (a sequence in which each nucleotide consists of only one nucleotide selected from four kinds of nucleotides) consists of at least three nucleotides, preferably seven or more nucleotides because it needs to anneal to a template nucleic acid. A portion of random combination, N (a mixture of G, A, T and C), may be included in a defined nucleotide sequence, for example, on the 3'-terminal side, on the 5'-terminal side or in the internal portion although there is no specific limitation concerning the position thereof. The random nucleotide sequence is preferably of 0 to 5 nucleotide(s). A nucleotide in defined nucleotide sequences of primers in a pool may be fixed to A, G, C or T. For example, in case of defined nucleotide sequences each consisting of seven nucleotides, the first and seventh nucleotides from the 3' terminus may be fixed to one of A, G, C and T. The GC content of the nucleotide sequence is preferably from 50% to 70%. For example, four or five nucleotides may be G or C in a defined nucleotide sequence of seven nucleotides. In this case, the nucleotide sequence is preferably determined such that the primer does not assume a secondary structure by itself or form a primer dimer.

A single band can be efficiently generated in a subsequent PCR by making a specific sequence for annealing to a template in a primer having a defined nucleotide sequence be of three or more nucleotides, more preferably seven or more nucleotides. The primer may contain a portion of a random nucleotide sequence. In particular, it is important to include a tag sequence in the primer.

According to the present invention, a pool of primers having the structure represented by General Formula above and defined nucleotide sequences can be used to generate substantially single-banded amplified fragments in subsequent PCRs and to obtain amplified fragments of varying lengths. Then, the entire nucleotide sequence of the template nucleic acid can be determined by analyzing the nucleotide sequences of the amplified fragments.

A pool of primers in which the nucleotide sequences specific for a template is of seven nucleotides exemplifies one embodiment of the pool of primers of the present invention. Although it is not intended to limit the present invention, examples thereof include the pools of primers I-III as described in Example 1. Example are as follows: the pool of primers I, IV or VI in which each primer contains a random nucleotide sequence on the 5'-terminal side of its template-specific nucleotide sequence; the pool of primers II in which each primer contains a random nucleotide sequence on the 3'-terminal side; and the pool of primers III or V without a portion of a random nucleotide sequence in the primers.

For example, in case where a template-specific nucleotide sequence is of seven nucleotides, it is preferable that six or more out of seven nucleotides of a defined nucleotide sequence in the primer anneal to a template nucleic acid for specific annealing. Although it is not intended to limit the present invention, for example, if defined nucleotide sequences are on the 3'-terminal sides, the variation of sequences of six nucleotides at the 3' termini of the defined sequences is particularly important, and it is preferable that two or more out of six nucleotides differ among the primers in pool.

Single-banded amplified fragments of varying sizes can be obtained in at least 10% of the total reactions by carrying out PCRs using combinations of a template-specific primer and primers in the pool of primers of the present invention. The entire nucleotide sequence of the nucleic acid of interest can be determined by subjecting the amplified fragments to direct sequencing.

The pool of primers of the present invention can be synthesized such that the primers have portions of defined nucleotide sequences, a tag sequence and random nucleotide sequences, for example, using the 394 type DNA synthesizer from Applied Biosystems Inc. (ABI) according to a phosphoramidite method. Alternatively, any methods including a phosphate triester method, an H-phosphonate method and a thiophosphonate method may be used to synthesize the pool of primers.

### (2) The method for determining a nucleotide sequence of the present invention

The method of the present invention is carried out by conducting PCRs using combinations of primers from the pool as described in (1) above and a template-specific primer, and determining the nucleotide sequences of the resulting amplified fragments.

A pol I-type, α-type or non-pol I, non-α-type DNA polymerase, or a mixture of DNA polymerases can be used as a DNA polymerase in a PCR according to the method of the present invention. Although it is not intended to limit the present invention, for example, Taq DNA polymerase (pol I-type), or KOD DNA polymerase or Pfu DNA polymerase (α-type) can be preferably used. In addition, a mixture of DNA polymerases may be used as a DNA polymerase. For example, a combination of one with a 3'→5' exo activity and one without a 3'→5' exo activity such as TaKaRa ExTaq DNA polymerase, TaKaRa LA-Taq DNA polymerase, TaKaRa Z-Taq DNA polymerase or KOD dash DNA polymerase can be preferably used. Furthermore, a combination of ones with 3'→5' exo activities as described in WO 99/54455 or ones without a 3'→5' exo activity may be preferably used in the method of the present invention.

dNTPs used for a PCR or the like (a mixture of dATP, dCTP, dGTP and dTTP) can be preferably used as nucleotide triphosphates that serve as substrates in an extension reaction in the method. The dNTPs may contain a dNTP analog such as 7-deaza-dGTP or the like as long as it serves as a substrate for the DNA polymerase used.

Amplified fragments of varying lengths starting from a template-specific primer as a basic point can be obtained in the method of the present invention by carrying out PCRs using a nucleic acid as a template, primers from the pool as described in (1) above and the template-specific primer in combination. Then, the entire nucleotide sequence of the template nucleic acid can be analyzed by subjecting the amplified fragments to sequencing.

According to the method of the present invention, a nucleic acid as a template may be a genome of an organism. A fragment obtained by cleaving a genome by a physical means or by digestion with a restriction enzyme, or a plasmid, phage, phagemid, cosmid, BAC or YAC vector having such a fragment being inserted can be preferably used as a template nucleic acid. Alternatively, it may be a cDNA obtained by a reverse transcription reaction.

A nucleic acid (DNA or RNA) used as a template in the method of the present invention may be prepared or isolated from any sample that may contain the nucleic acid. Alternatively, such a sample may be used directly in the nucleic acid amplification reaction according to the present invention. Examples of the samples that may contain the nucleic acid include, but are not limited to, samples from organisms such as a whole blood, a serum, a buffy coat, a urine, feces, a cerebrospinal fluid, a seminal fluid, a saliva, a tissue (e.g., a cancerous tissue or a lymph node) and a cell culture (e.g., a mammalian cell culture or a bacterial cell culture), samples that contain a nucleic acid such as a viroid, a virus, a bacterium, a fungi, a yeast, a plant and an animal, samples suspected to be contaminated or infected with a microorganism such as a virus or a bacterium (e.g., a food or a biological formulation), and samples that may contain an organism such as a soil and a waste water. The sample may be a preparation containing a nucleic acid obtained by processing a sample as described above according to a known method. Examples of preparations that can be used in the present invention include a cell destruction product or a sample obtained by fractionating the product, a nucleic acid in the sample, or a sample in which specific nucleic acid molecules such as mRNAs are enriched. Furthermore, a nucleic acid such as a DNA or an RNA obtained amplifying a nucleic acid contained in a sample by a known method can be preferably used.

A preparation containing a nucleic acid can be prepared from a material as described above by using, for example, lysis with a detergent, sonication, shaking/stirring using glass beads or a French press, without limitation. In some cases, it is advantageous to further process the preparation to purify the nucleic acid (e.g., in case where an endogenous nuclease exists). In such cases, the nucleic acid is purified by a known means such as phenol extraction, chromatography, ion exchange, gel electrophoresis or density-gradient centrifugation.

The method of the present invention may comprise selecting a pool of primers to be used depending on the origin of a nucleic acid as a template.

When it is desired to use a nucleic acid having a sequence derived from an RNA as a template, the method of the present invention may be conducted using, as a template, a cDNA synthesized by a reverse transcription reaction in which the RNA is used as a template. Any RNA for which one can make a primer to be used in a reverse transcription reaction can be applied to the method of the present invention, including total RNA in a sample, RNA molecules such as mRNA, tRNA and rRNA as well as specific RNA molecular species.

Any primer that anneals to an RNA as a template under reaction conditions used may be used in a reverse transcription reaction. The primer may be a primer having a nucleotide sequence that is complementary to a specific RNA as a template (a specific primer), an oligo-dT (deoxythymine) primer and a primer having a random sequence (a random primer). In view of specific annealing, the length of the primer for reverse transcription is preferably 6 nucleotides or more, more preferably 9 nucleotides or more. In view of oligonucleotide synthesis, the length is preferably 100 nucleotides or less, more preferably 30 nucleotides or less.

Any enzyme that has an activity of synthesizing a cDNA using an RNA as a template can be used in a reverse transcription reaction. Examples thereof include reverse transcriptases originating from various sources such as avian myeloblastosis virus-derived reverse transcriptase (AMV RTase), Molony murine leukemia virus-derived reverse transcriptase (MMLV RTase) and Rous-associated virus 2 reverse transcriptase (RAV-2 RTase). In addition, a DNA polymerase that also has a reverse transcription activity can be used. An enzyme having a reverse transcription activity at a high temperature such as a DNA polymerase from a bacterium of genus Thermus (e.g., Tth (Thermus thermophilus) DNA polymerase) and a DNA polymerase from a thermophilic bacterium of genus Bacillus is preferable for the present invention. For example, DNA polymerases from thermophilic bacteria of genus Bacillus such as a DNA polymerase from B. st (Bacillus stearothermophilus) (Bst DNA polymerase) and a DNA polymerase from Bca (Bacillus cardotenax) are preferable, although it is not intended to limit the present invention. For example, Bca DNA polymerase does not require a manganese ion for the reverse transcription reaction. Furthermore, it can synthesize a cDNA while suppressing the formation of a secondary structure of an RNA as a template under high-temperature conditions. Both a naturally occurring one and a variant of the above-mentioned enzyme having a reverse transcriptase activity can be used as long as they have the activity.

According to the method of the present invention, a PCR can be carried out, for example, using a reaction consisting of three steps. The three steps are a step of dissociating (denaturing) a double-stranded DNA into single-stranded DNAs, a step of annealing a primer to the single-stranded DNA and a step of synthesizing (extending) a complementary strand from the primer in order to amplify a region of a DNA of interest. Alternatively, it may be conducted using a reaction designated as "the shuttle PCR" ("PCR hou saizensen" (Recent advances in PCR methodology), Tanpakushitsu Kakusan Kouso, Bessatsu, (Protein, Nucleic Acid and Enzyme, Supplement), 41(5):425-428 (1996)) in which two of the three steps, that is, the step of annealing the primer and the step of extending are carried out at the same temperature. In addition, the conditions for the PCR according to the method of the present invention may be the conditions for the high-speed PCR method as described in WO 00/14218. The reaction mixture may contain an acidic substance or a cationic complex as described in WO 99/54455.

A nucleotide sequence of an amplified DNA fragment obtained by a PCR as described above can be determined by subjecting the DNA fragment to an appropriate procedure for determining a nucleotide sequence of a DNA such as a chain terminator method. By totally analyzing similarly determined nucleotide sequences of respective PCR-amplified fragments, a nucleotide sequence of a wide region in the nucleic acid as a template can be determined.

According to the method of the present invention, a PCR product may be subjected to sequencing after it is purified by subjecting it to an appropriate means of purification such as a molecular sieve for purifying a PCR product (e.g., Microcon-100).

In an exemplary nucleotide sequence analysis using the method of the present invention in which a genome of Escherichia coli is analyzed, single-banded PCR-amplified products are obtained in 22 out of 92 reactions using the pool of primers of the present invention which contains 92 primers each having a tag sequence. By subjecting the amplified fragments to direct sequencing, a nucleotide sequence of about 4,000 bp or more can be determined. In case of a genome of Pyrococcus furiosus, single-banded PCR-amplified fragments are obtained in 18 out of 92 reactions, and a nucleotide sequence can be determined over a region of about 5,000 bp or more. In case of a genome of Bacillus cardotenax, by mixing plasmids having a DNA fragment derived from the genome inserted in different directions, single-banded PCR-amplification products are obtained in 20 out of 92 reactions (total for both directions), and a nucleotide sequence can be determined in both directions over a region of about 2,000 bp or more.

A DNA fragment amplified by a PCR as described above has a tag sequence derived from a primer selected from a pool of primers at its terminus. Thus, the nucleotide sequence of the amplified DNA fragment can be determined by using a primer having the same nucleotide sequence as the tag sequence.

According to the present invention, a nucleotide sequence is determined by direct sequencing. As used herein, direct sequencing refers to determination of a nucleotide sequence of a nucleic acid using an amplified nucleic acid fragment as a template without cloning it into a vector. Direct sequencing is carried out according to a conventional method for determining a nucleotide sequence (e.g., a dideoxy method) using a fragment obtained by an amplification method (e.g., a PCR) as a template and a primer having a sequence complementary to the fragment, for example, a primer having the same nucleotide sequence as a tag sequence.

The whole amplified fragment obtained in a PCR as described above is subjected to a sequencing reaction. For a reaction resulting in a substantially single-banded PCR-amplified fragment, the nucleotide sequence of the PCR-amplified fragment is determined even if a background due to amplified fragments consisting of primers is observed in the reaction. As used herein, a substantially single-banded amplified fragment refers to an amplified fragment that is so single that it enables an analysis of the nucleotide sequence thereof in a subsequent sequence analysis. In the method of the present invention, any nucleic acid amplification method that can be used to obtain a substantially single-banded amplified fragment can be preferably used. Examples thereof include, but are not limited to, the PCR, the ICAN, the SDA or the RCA. The entire nucleotide sequence of the original template nucleic acid can be determined by totally analyzing the results. It may be impossible to determine apart of a nucleotide sequence of a template nucleic acid, for example, because of nonuniform PCR amplification of regions. In this case, it is natural to carry out the method of the present invention for the region again, or to determine the nucleotide sequence of the region using a known method in combination, for example, using a primer newly synthesized based on the obtained nucleotide sequence information or the like.

A commercially available sequencer such as Mega BACE 11000 (Amersham Pharmacia Biotech), a commercially available sequencing kit such as BcaBEST™ Dideoxy Sequencing Kit (Takara Shuzo) or the like may be used for nucleotide sequence determination.

In a preferable embodiment, although it is not intended to limit the present invention, a PCR amplification product is subjected to agarose gel electrophoresis or the like to analyze the amplification product, reactions resulting in substantially single-banded amplified fragments and reactions resulting in products of suitable lengths for the nucleotide sequence determination method of the present invention are selected, and then the reaction products are subjected to sequencing. By including the above-mentioned steps, the number of amplified fragments to be subjected to sequencing can be decreased to reduce the cost required for nucleotide sequence determination. Furthermore, if reactions resulting in substantially single bands are selected, the molecule (mole) number of the amplified fragment of interest is sufficiently greater than those of other amplified fragments. As a result, reliable sequence data with little noise can be obtained even if a sequencing reaction is carried out utilizing a tag sequence. It is important to estimate the amount of an amplified fragment after converting it into the number of molecules in order to select a reaction resulting in a substantially single-banded amplified fragment. Although it is not intended to limit the present invention, for example, electrophoresis equipment of Agilent 2100 Bioanalyzer (Takara Shuzo) can be effectively utilized. Using the equipment, the amount and the molecular weight of an amplified fragment can be determined, and the amount of the fragment can be expressed after converting it into the number of molecules based on the determined values. In view of economical efficiency of sequence determination, it is important to select reactions resulting in products with appropriate lengths in order to obtain nucleotide sequence data distributed as uniformly as possible with little overlap over the entire region of which the nucleotide sequence is to be determined. The labor and time required for nucleotide sequence determination can be greatly reduced by constructing a system using a computer by which the above-mentioned two selection steps automated.

Furthermore, if multiple amplified fragments are observed upon an analysis of an amplification product, one of the amplified fragments (for example, the most abundant amplified fragment) can be isolated according to a known method and subjected to sequencing.

In some cases, a band corresponding to a size of a product resulting from amplification utilizing only a primer specific for a known sequence may be generated in all reactions when PCRs are carried out using all the primers in a pool and the specific primer. Since such an amplified fragment does not contain a tag sequence, a nucleotide sequence can be determined even if an amplified fragment of interest is contaminated with such a fragment as a background. Nevertheless, since the amplification utilizing only the specific primer reduces the amplification efficiency of a nucleic acid of interest, it is preferable to design a primer sequence such that such amplification does not occur. Although it is not intended to limit the present invention and it depends on the template sequence, it is generally preferable that the 3' terminus of a primer is AT-rich.

The pool of primers used in the method of the present invention is a pool containing the primers as described in (1) above. PCRs are independently carried out using each of the primers and a primer specific for a known sequence in a template nucleic acid in combination. Although it is not intended to limit the present invention, if a defined nucleotide sequence of a primer in a pool is of seven nucleotides, such a sequence appears at an average frequency of one in 4⁷ (= 16384) provided that the nucleotide distribution in the sequence is completely uniform. If so, a sequence in 100 kinds of defined nucleotide sequences of seven nucleotides in primers appears at an average frequency of one in about 160 nucleotides. Thus, amplified fragments of which the lengths differ each other by 160 nucleotides on the average are obtained by carrying out PCRs independently using such 100 primers. Among these, substantially single-banded PCR products are subjected to sequencing reactions using a primer for sequencing having the same nucleotide sequence as the tag sequence or a nucleotide sequence contained in the tag sequence. The thus obtained nucleotide sequence data are analyzed. Thereby, a sequence of several kilobases can be analyzed at once without awaiting subsequently obtained sequence data.

### (3) The kit containing the pool of primers of the present invention

The present invention provides a kit for carrying out the method for determining a nucleotide sequence of a nucleic acid as described in (2) above using the pool of primers as described in (1) above. In one embodiment, the kit is in a packed form and contains specifications of the pool of primers of the present invention and instructions for a PCR using the pool. A kit containing the pool of primers of the present invention, a DNA polymerase and a buffer for the polymerase can be preferably used for the method of the present invention. Alternatively, the pool of primers of the present invention, a commercially available DNA polymerase and a reagent for a PCR may be selected according to instructions and then used. The kit may contain a reagent for a reverse transcription reaction for a case where an RNA is used as a template. A DNA polymerase can be selected from the DNA polymerases used according to the present invention as described in (2) above. A commercially available reagent for a PCR may be used as a reagent for a PCR, and the buffers as described in Examples may be used. Furthermore, the kit may contain a reagent for nucleotide sequence determination such as a primer or a polymerase for sequencing.

Instructions describing the nucleotide sequence determination method of the present invention provide a third party with information on the nucleotide sequence determination method of the present invention, the method of using the kit, specifications of a recommended pool of primers, recommended reaction conditions and the like. The instructions include printed matters describing the above-mentioned contents such as an instruction manual in a form of a pamphlet or a leaflet, a label stuck to the kit, and description on the surface of the package containing the kit. The instructions also include information disclosed or provided through electronic media such as the Internet.

### (4) The composition of the present invention

The present invention provides a composition used for the above-mentioned method for determining a nucleotide sequence of a nucleic acid. An exemplary composition contains the pool of primers as described in (1) above and the DNA polymerase as described in (2) above. The composition may further contain a buffering component, a magnesium salt, dNTP or the like as a component for carrying out a PCR. Furthermore, the composition may contain an acidic substance or a cationic complex as described in (2) above.

By using the pool of primers of the present invention, a rapid and low-cost method for determining a nucleotide sequence of a nucleic acid is provided. Since the method can be carried out using a pool of primers containing about 100 primers and one specific primer in combination, it is useful for analyses of large amounts and many kinds of genomes. Furthermore, the method of the present invention is useful for analyses of large amounts and many kinds of genomes also because a nucleotide sequence of a nucleic acid of interest can be determined with fewer sequencing procedures than those required for a conventional shotgun sequencing method.

### Examples

The following Examples illustrate the present invention in more detail, but are not to be construed to limit the scope thereof.

### Example 1

### (1) Construction of pool of primers I

Primers each containing the nucleotide sequence of SEQ ID NO:1 GGCACGATTCGATAACG as a tag sequence were synthesized. In other words, a pool of primers I represented by General Formula (I) was synthesized:
5'-tag sequence-NN-SSSSSSS-3' (I)
(N: a mixture of G, A, T and C; S: a defined nucleotide selected from G, A, T or C).

The structure of the pool of primers I and the defined nucleotide sequences represented by SSSSSSS are shown in Table 1.

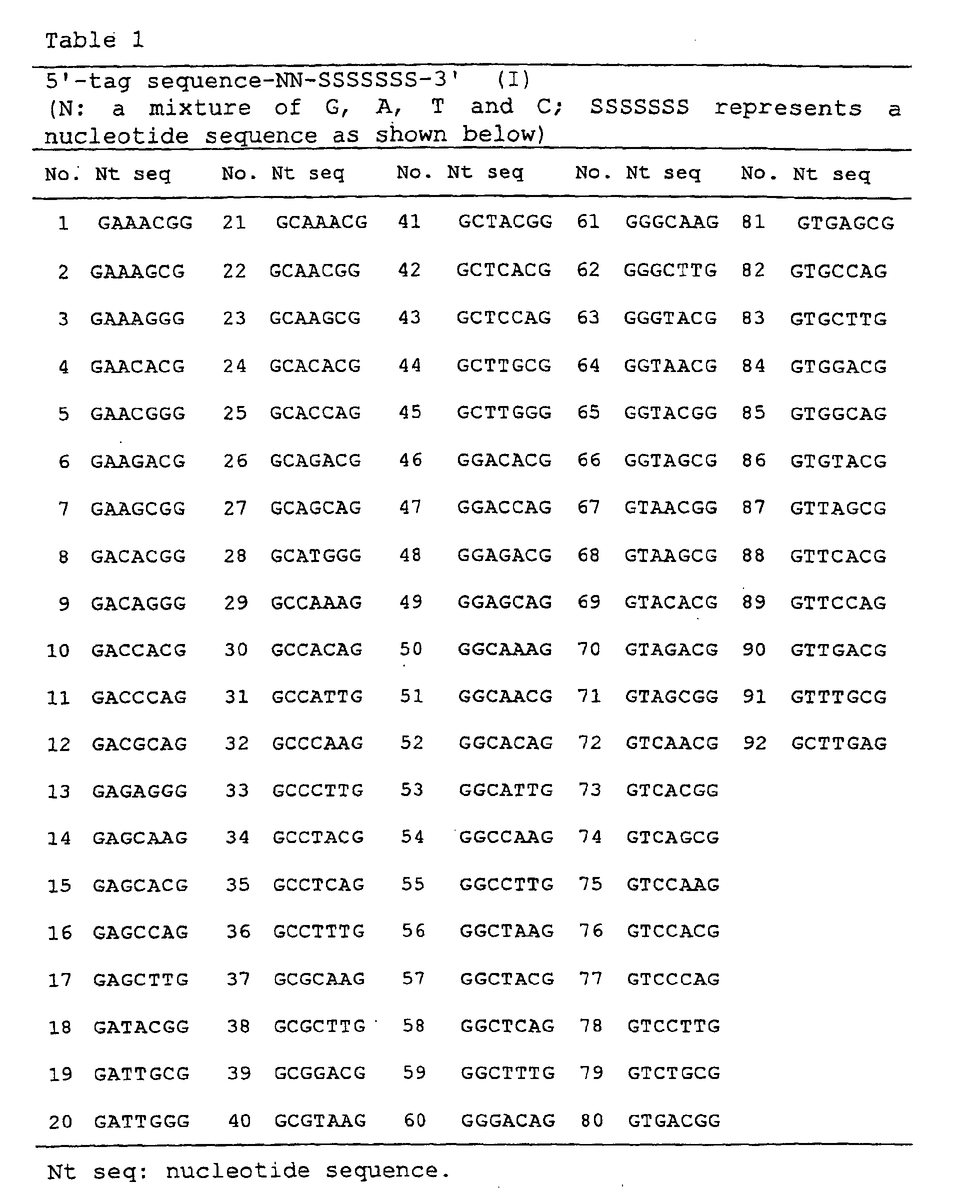

Table 1 shows defined nucleotide sequences of seven nucleotides at the 3' termini of primers represented by General Formula I. 92 defined nucleotide sequences were selected for the primers from 4⁷ (= 16384) nucleotide sequences taking the Tm values, the secondary structures of the primers and the possibilities of primer dimer formation into consideration.

### (2) Construction of pool of primers II

Primers each containing the nucleotide sequence of SEQ ID NO:2 GGCACGATTCGATAAC as a tag sequence were synthesized. In other words, a pool of primers II represented by General Formula (II) was synthesized:
5'-tag sequence-SSSSSSS-NN-3' (II)
(N: a mixture of G, A, T and C; S: a defined nucleotide selected from G, A, T or C).

The structure of the pool of primers II and the defined nucleotide sequences represented by SSSSSSS are shown in Table 2.

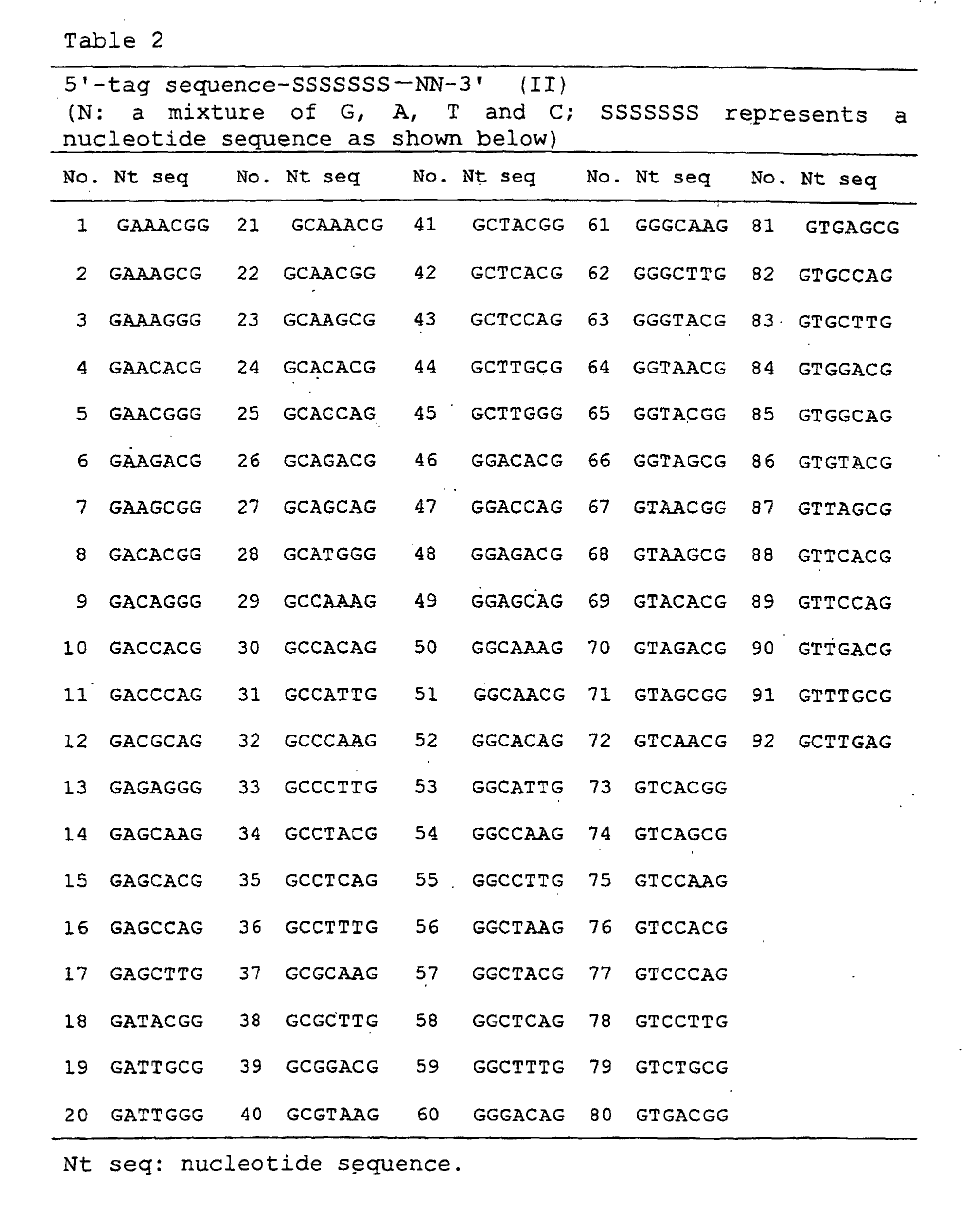

Table 2 shows defined nucleotide sequences of the third to ninth nucleotides at the 3' termini of primers represented by General Formula II. 92 defined nucleotide sequences were selected for the primers from 4⁷ (= 16384) nucleotide sequences taking the Tm values, the secondary structures of the primers and the possibilities of primer dimer formation into consideration.

### (3) Construction of pool of primers III

Primers each containing the nucleotide sequence of SEQ ID NO:2 GGCACGATTCGATAAC as a tag sequence were synthesized. In other words, a pool of primers III represented by General Formula (III) was synthesized:
5'-tag sequence-SSSSSSS-3' (III)
(S: a defined nucleotide selected from G, A, T or C).

The structure of the pool of primers III and the defined nucleotide sequences represented by SSSSSSS are shown in Table 3.

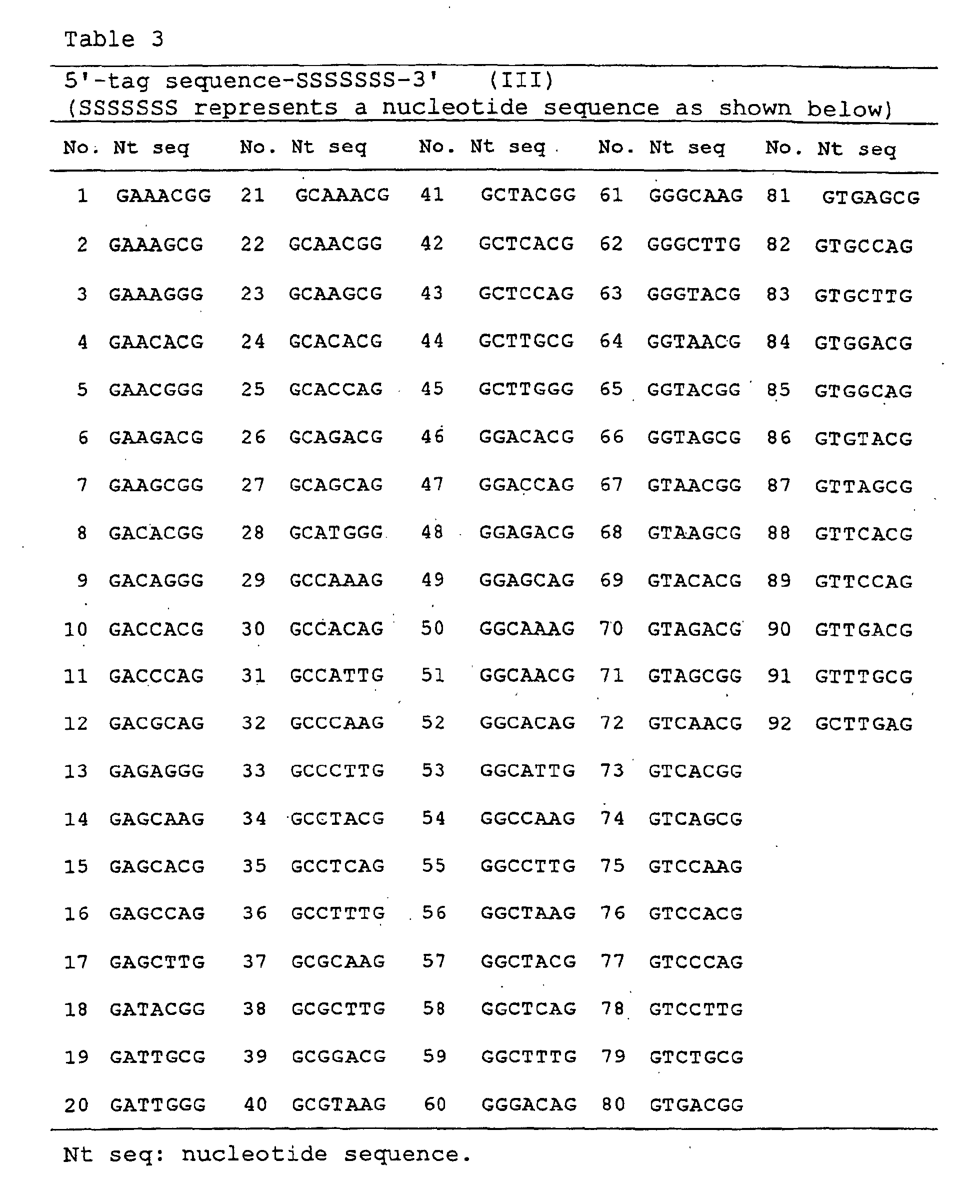

Table 3 shows defined nucleotide sequences of seven nucleotides at the 3' termini of primers represented by General Formula III. 92 defined nucleotide sequences were selected for the primers from 4⁷ (= 16384) nucleotide sequences taking the Tm values, the secondary structures of the primers and the possibilities of primer dimer formation into consideration.

### (4) Construction of pool of primers IV

Primers each containing the nucleotide sequence of SEQ ID NO:3 CAGGAAACAGCTATGAC as a tag sequence were synthesized. In other words, a pool of primers IV represented by General Formula (IV) was synthesized:
5'-tag sequence-NNN-SSSSSS-3' (IV)
(N: a mixture of G, A, T and C; S: a defined nucleotide selected from G, A, T or C).

The structure of the pool of primers IV and the defined nucleotide sequences represented by SSSSSS are shown in Table 4.

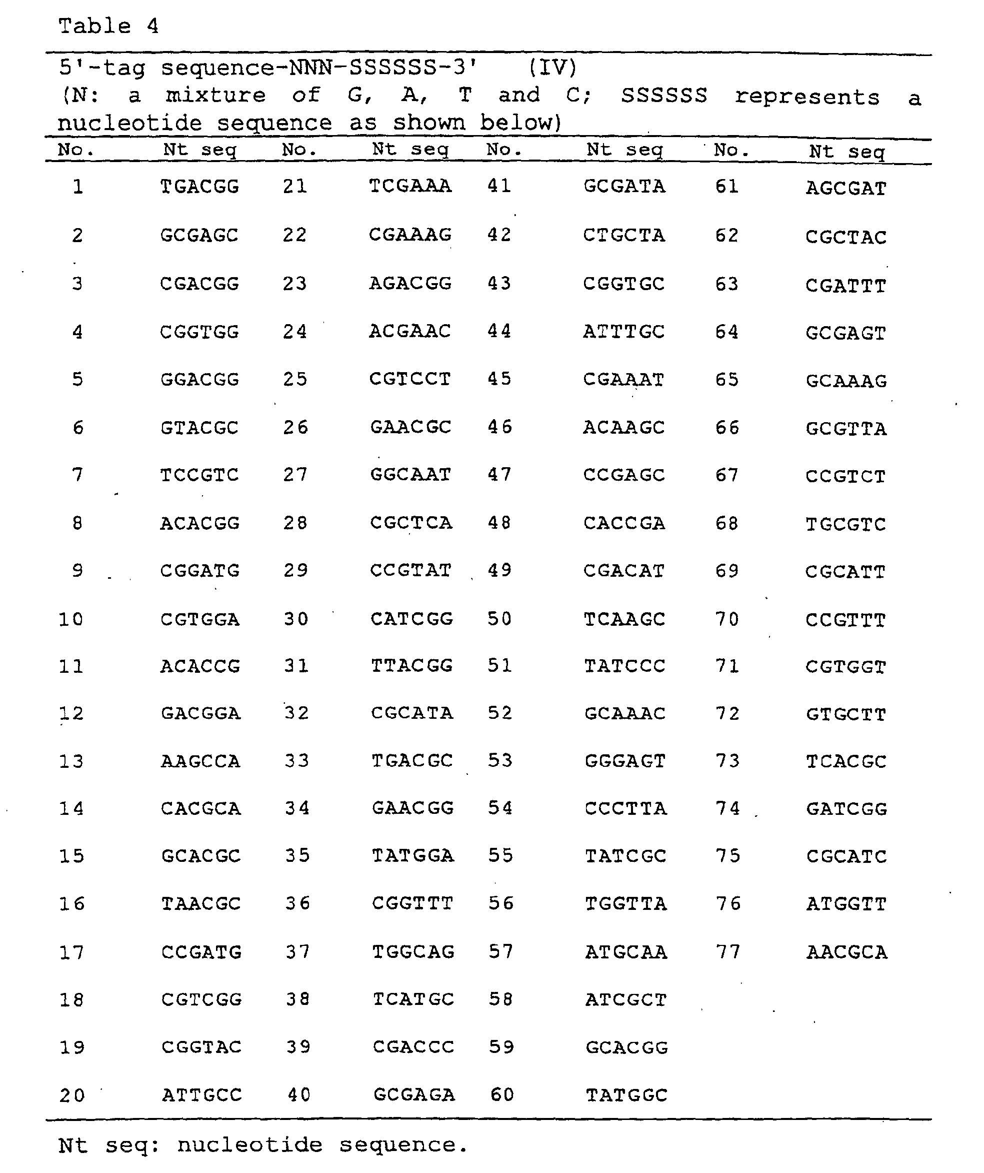

Table 4 shows defined nucleotide sequences of six nucleotides at the 3' termini of primers represented by General Formula IV. 77 defined nucleotide sequences were selected for the primers from 4⁶ (= 4096) nucleotide sequences taking the Tm values, the secondary structures of the primers and the possibilities of primer dimer formation into consideration.

### (5) Construction of pool of primers V

Primers each containing the nucleotide sequence of SEQ ID NO:3 CAGGAAACAGCTATGAC as a tag sequence were synthesized. In other words, a pool of primers V represented by General Formula (V) was synthesized:
5'-tag sequence-SSS-3' (V)
(S: a defined nucleotide selected from G, A, T or C).

The structure of the pool of primers V and the defined nucleotide sequences represented by SSS are shown in Table 5.

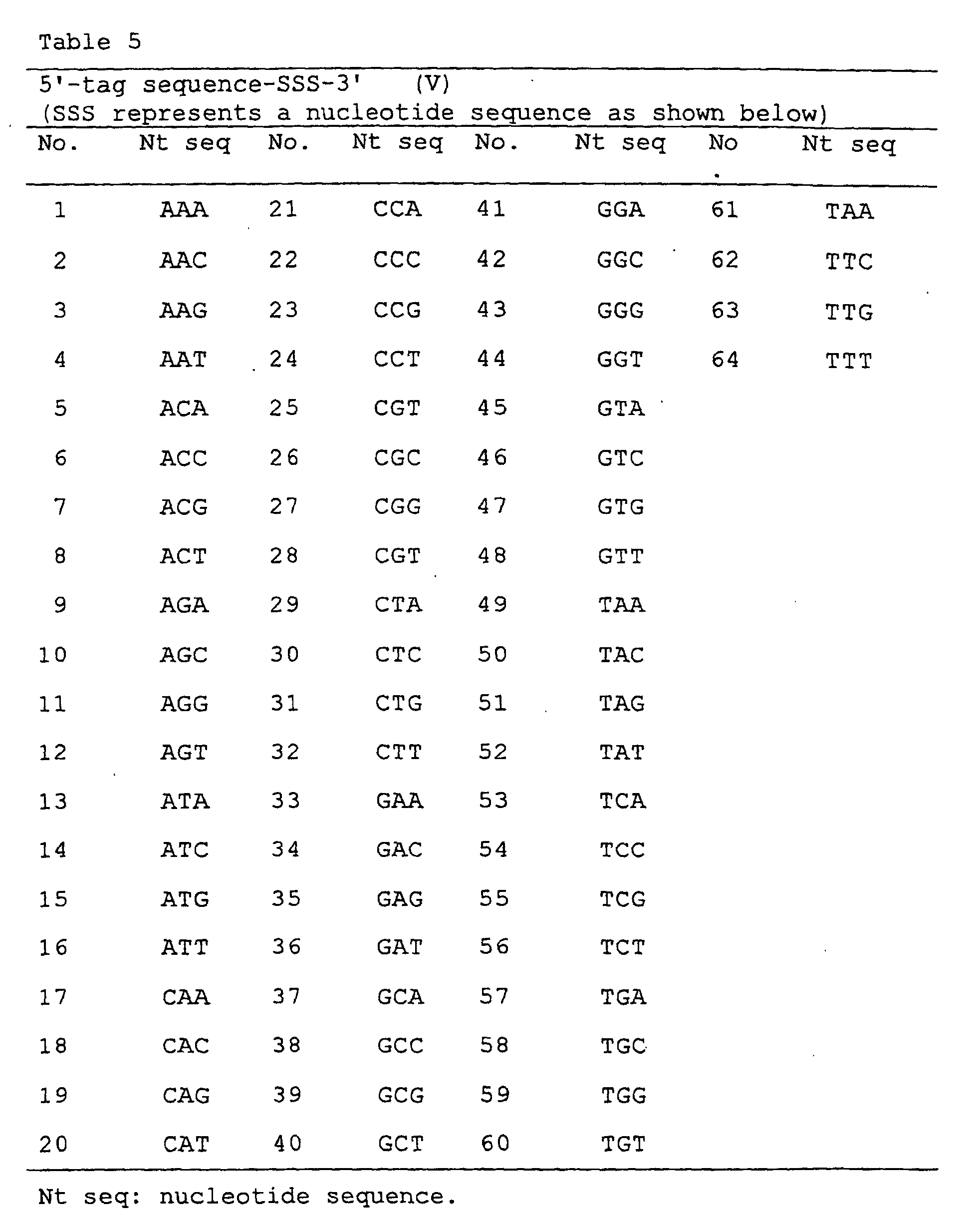

Table 5 shows nucleotide sequences of three nucleotides at the 3' termini of primers represented by General Formula V. 4³ (= 64) nucleotide sequences were selected for the primers.

### Example 2

(1) A method for determining a nucleotide sequence of an Escherichia coli gene cloned into a plasmid was examined. A plasmid clone was prepared as follows. Briefly, a PCR was carried out using a genomic DNA from Escherichia coli JM109 (Takara Shuzo) as a template and primers Eco-1 and E6sph having nucleotide sequences of SEQ ID NOS:4 and 5, respectively. The resulting PCR-amplified fragment of about 6.1 kbp was blunt-ended using TaKaRa Blunting Kit (Takara Shuzo), digested with a restriction enzyme SphI (Takara Shuzo) and ligated with a plasmid pUC119 (Takara Shuzo) between the SmaI and SphI sites to obtain a plasmid pUCE6.
(2) PCRs were carried out using the plasmid pUCE6 as a template, and a primer M13-primer RV (Takara Shuzo) which has a nucleotide sequence specific for the vector and each one of the primers in the pools of primers I to III prepared in Example 1 each containing 92 primers. 25 µl of a reaction mixture for a PCR containing 20 mM tris-acetate (pH 8.5), 50 mM potassium acetate, 3 mM magnesium acetate, 0.01% BSA, 300 µM each of dNTPs, 100 pg of the plasmid pUCE6, 0.625 units of TaKaRa ExTaq DNA polymerase (Takara Shuzo) was prepared. The reaction mixture was subjected to a PCR of 30 cycles each consisting of 98°C for 0 second, 38°C for 0 second and 72°C for 90 seconds using Gene Amp PCR system 9600 (Perkin Elmer). Then, 2 µl each of the reaction mixtures was subjected to electrophoresis on agarose gel, and amplified DNA fragments were observed after staining with ethidium bromide.
   Single-banded PCR-amplified fragments of varying sizes ranging from 300 bp to 5600 bp were obtained in 22 out of 92 reactions using the pool of primers I. The amplified fragments were subjected to removal of primers and salts from the reaction mixtures using Microcon-100 (Takara Shuzo), and direct sequencing using a sequencing primer having a nucleotide sequence of SEQ ID NO:2 (the tag sequence) according to a conventional method. As a result, a sequence of 4378 nucleotides in the DNA fragment inserted into the plasmid pUCE6 could be determined. Single PCR-amplified DNA fragments of varying sizes ranging from 300 bp to 4700 bp were obtained in 21 out of 92 reactions using the pool of primers II. The amplified fragments were subjected to direct sequencing, and a sequence of 4601 nucleotides could be determined. Single-banded PCR-amplified DNA fragments of varying sizes ranging from 1000 bp to 6000 bp were obtained in 24 out of 92 reactions using the pool of primers III. The amplified fragments were subjected to direct sequencing, and the nucleotide sequence of the template nucleic acid could also be determined as described above for other pools of primers.
(3) Use of a commercially available ExTaq buffer in a PCR was also examined. The composition of the reaction mixture for a PCR was the same as that as described in (2) above except that a buffer for TaKaRa Ex-Taq DNA polymerase (Takara Shuzo) and 200 µM each of dNTPs were used. The reaction mixture was subjected to a PCR of 30 cycles each consisting of 98°C for 0 second, 38°C for 0 second and 72°C for 3 minutes using Gene Amp PCR system 9600 (Perkin Elmer). Then, 2 µl each of the reaction mixtures was subjected to electrophoresis on agarose gel, and amplified DNA fragments were observed after staining with ethidium bromide. As a result, results similar to those as described in (2) above were obtained for the respective pools of primers.
(4) The mode of annealing of a primer in the method of the present invention was examined. In case of the pool of primers I, single-banded PCR products were obtained in 22 out of 92 reactions, and then the nucleotide sequence of the template nucleic acid could be determined. In 12 reactions, the defined nucleotide sequences of seven nucleotides in the primers matched completely with the template nucleic acid. In 10 reactions among the 22 reactions, the annealings involved mismatches of one nucleotide. An identical region was amplified in 5 out of the 10 reactions, and another identical region was amplified in 2 out of the 10 reactions.

In case of the pool of primers II, single-banded PCR products were obtained in 21 out of 92 reactions, and then the nucleotide sequence of the template nucleic acid could be determined. In 9 reactions, the defined nucleotide sequences of seven nucleotides in the primers matched completely with the template nucleic acid. In 10 out of 92 reactions, the annealings involved mismatches of one nucleotide. In 2 out of the 92 reactions, the annealings involved mismatches of two nucleotides.

### Example 3

(1) A method for determining a nucleotide sequence of a Pyrococcus furiosus gene with a low GC content (43.2%) cloned into a plasmid was examined. A plasmid clone was prepared as follows. Briefly, a PCR was carried out using a genomic DNA from Pyrococcus furiosus (DSM accession no. 3638) as a template and primers PfuFXba and PfuRXba having nucleotide sequences of SEQ ID NOS:6 and 7, respectively. The resulting PCR-amplified fragment of about 8.5 kbp was digested with a restriction enzyme XbaI (Takara Shuzo) and ligated with a plasmid pTV119N (Takara Shuzo) at the XbaI site to obtain a plasmid pTVPfu8.5.
(2) PCRs were carried out using the plasmid pTVPfu8.5 as a template, and a primer MR1 which has a nucleotide sequence specific for the vector (SEQ ID NO:8) and each one of the 92 primers in the pool of primers I prepared in Example 1. 100 µl of a reaction mixture for a PCR containing 20 mM tris-acetate (pH 8.5), 50 mM potassium acetate, 3 mM magnesium acetate, 0.01% BSA, 300 µM each of dNTPs, 200 pg of the plasmid pTVPfu8.5, 2.5 units of TaKaRa ExTaq DNA polymerase was prepared. The reaction mixture was subjected to a PCR of 30 cycles each consisting of 98°C for 10 seconds, 38°C for 10 seconds and 72°C for 2 minutes using Gene Amp PCR system 9600. Then, 2 µl each of the reaction mixtures was subjected to electrophoresis on agarose gel, and amplified DNA fragments were observed after staining with ethidium bromide.
   Single PCR-amplified fragments of varying sizes ranging from 1300 bp to 8400 bp were obtained in 18 out of 92 reactions. The amplified fragments were subjected to removal of primers and salts from the reaction mixtures using Microcon-100 (Takara Shuzo), and direct sequencing using a sequencing primer having a nucleotide sequence of SEQ ID NO:2 (the tag sequence) according to a conventional method. As a result, a sequence of 5622 nucleotides in the DNA fragment inserted into the plasmid pTVPfu8.5 could be determined.
(3) Use of a commercially available ExTaq buffer in a PCR was also examined. The composition of the reaction mixture for a PCR was the same as that as described in (2) above except that a buffer for TaKaRa Ex-Taq DNA polymerase (Takara Shuzo) and 200 µM each of dNTPs were used. The reaction mixture was subjected to a PCR of 30 cycles each consisting of 98°C for 10 seconds, 38°C for 10 seconds and 72°C for 4 minutes using Gene Amp PCR system 9600. Then, 2 µl each of the reaction mixtures was subjected to electrophoresis on agarose gel, and amplified DNA fragments were observed after staining with ethidium bromide. As a result, results similar to those as described in (2) above were obtained for the pool of primers.
(4) The mode of annealing of a primer in the method of the present invention was examined. In case of the pool of primers I, single-banded PCR products were obtained in 19 out of 92 reactions, and then the nucleotide sequence of the template nucleic acid could be determined. In 4 reactions, the defined nucleotide sequences of seven nucleotides in the primers matched completely with the template nucleic acid. In 9, 1 and 1 reaction(s) among the 92 reactions, the annealings involved mismatches of one nucleotide, two nucleotides and three nucleotides, respectively. Annealing to the identical sequence was observed in 2 reactions with one nucleotide-mismatched annealing.

### Example 4

(1) A method for determining a nucleotide sequence of a Bacillus cardotenax gene having many repeats of GC clusters and AT clusters cloned into a plasmid was examined. A plasmid clone was prepared as follows. Briefly, a genomic DNA from Bacillus cardotenax (DSM accession no. 406) was digested with a restriction enzyme HindIII (Takara Shuzo) and ligated with a plasmid pUC118 (Takara Shuzo) at the HindIII site to obtain a plasmid pUCBcaF2.7 having an inserted DNA fragment of 2.7 kbp. In addition, a plasmid pUCBcaR2.7 having the DNA fragment inserted in the opposite direction was obtained.
(2) PCRs were carried out using a mixture of the plasmids pUCBcaF2.7 and pUCBcaR2.7 as a template, and a primer M13-primer RV which has a nucleotide sequence specific for the vector and each one of the 92 primers in the pool of primers I prepared in Example 1. 100 µl of a reaction mixture for a PCR containing 20 mM tris-acetate (pH 8.5), 50 mM potassium acetate, 3 mM magnesium acetate, 0.01% BSA, 300 µM each of dNTPs, 200 pg of a mixture of the plasmids pUCBcaF2.7 and pUCBcaR2.7, 2.5 units of TaKaRa ExTaq DNA polymerase was prepared. The reaction mixture was subjected to a PCR of 30 cycles each consisting of 98°C for 10 seconds, 38°C for 10 seconds and 72°C for 2 minutes using Gene Amp PCR system 9600. Then, 2 µl each of the reaction mixtures was subjected to electrophoresis on agarose gel, and amplified DNA fragments were observed after staining with ethidium bromide.
   Single PCR-amplified fragments of varying sizes ranging from 650 bp to 2800 bp were obtained in 19 out of 92 reactions. The amplified fragments were subjected to removal of primers and salts from the reaction mixtures using Microcon-100, and direct sequencing using a sequencing primer having a nucleotide sequence of SEQ ID NO:2 (the tag sequence) according to a conventional method. As a result, a sequence of 2254 nucleotides in the DNA fragment inserted into the plasmids could be determined in both directions.
(3) Use of a commercially available ExTaq buffer in a PCR was also examined. The composition of the reaction mixture for a PCR was the same as that as described in (2) above except that a buffer for TaKaRa Ex-Taq DNA polymerase (Takara Shuzo) and 200 µM each of dNTPs were used. The reaction mixture was subjected to a PCR of 30 cycles each consisting of 98°C for 10 seconds, 38°C for 10 seconds and 72°C for 4 minutes using Gene Amp PCR system 9600. Then, 2 µl each of the reaction mixtures was subjected to electrophoresis on agarose gel, and amplified DNA fragments were observed after staining with ethidium bromide. As a result, results similar to those as described in (2) above were obtained for the pool of primers.
(4) The mode of annealing of a primer in the method of the present invention was examined. - In case of the pool of primers I, single-banded PCR products were obtained in 19 out of 92 reactions, and then the nucleotide sequence of the template nucleic acid could be determined. In 6 reactions, the defined nucleotide sequences of seven nucleotides in the primers matched completely with the template nucleic acid. In 8 and 2 reactions, the annealings involved mismatches of one nucleotide and two nucleotides, respectively.

### Example 5

The mode of annealing of a primer in the method of the present invention was examined with respect to the results of Examples 2 to 4.

It was confirmed that PCR amplification resulting in a single band and sequencing could be carried out according to the method of the present invention even if the defined nucleotide sequence of seven nucleotides was not completely matched with the template nucleic acid. The positions of nucleotides in the seven nucleotides of the primers that were not complementary to the template were studied. For the 43 reactions with mismatch annealing that could be successful in sequencing, the numbers of reactions and the positions of the mismatches in the seven nucleotides (indicated in parentheses) were as follows; 5 (3' terminus); 3 (second from the 3' terminus); 2 (third from the 3' terminus); 6 (fourth from the 3' terminus); 6 (fifth from the 3' terminus); 5 (sixth from the 3' terminus); and 16 (seventh from the 3' terminus). In many cases, PCR amplification and sequencing could be carried out even if the seventh nucleotide from the 3' terminus was mismatched. Thus, it was shown that the variation at the seventh position from the 3' terminus of each primer in a pool might not be indispensable. On the other hand, single-banded amplification products could be obtained and sequencing could be carried out even if the mismatches were located at the 3' termini in five reactions. The types of mismatches are shown in Table 6.

**Table 6**

| Position | | Position of mismatch | | | | | |
|---|---|---|---|---|---|---|---|
| (from the 3' terminus) | | | | | | | |
| 3' terminus | G-T=4 | | | | G-A=1 | | |
| Second | G-T=1 | | T-T=1 | | | | C-A=1 |
| Third | | C-T=1 | T-T=1 | | | | |
| Fourth | G-T=1 | C-T=3 | T-T=1 | | G-A=1 | | |
| Fifth | G-T=1 | C-T=1 | | G-G=1 | G-A=1 | A-A=1 | C-A=1 |
| Sixth | G-T=1 | C-T=1 | | G-G=1 | G-A=1 | A-A=1 | |
| Seventh | G-T=11 | | | G-G=1 | G-A=4 | | |
| Total | G-T=19 | C-T=6 | T-T=3 | G-G=3 | G-A=8 | A-A=2 | C-A=2 |

As shown in Table 6, many of the mismatched pairs comprised T. Thus, it was confirmed that T tends to cause a mismatch at a higher frequency than other nucleotides.

### Example 6

(1) A method for determining a nucleotide sequence of a Pyrococcus furiosus gene cloned into a cosmid was examined. A cosmid 491 as described in WO 97/24444 into which a Pyrococcus furiosus gene of 40 kbp had been inserted was used as a cosmid clone. The examination was carried out as follows.
(2) PCRs were carried out using the cosmid 491 as a template, and a primer Pfu30F1 which has a nucleotide sequence specific for the insert (SEQ ID NO:9) and each one of the 92 primers from the pool of primers I, the 92 primers from the pool of primers II, the 77 primers from the pool of primers IV and the 64 primers from the pool of primers V prepared in Example 1. 100 µl of a reaction mixture for a PCR containing 20 mM tris-acetate (pH 8.5), 50 mM potassium acetate, 3 mM magnesium acetate, 0.01% BSA, 300 µM each of dNTPs, 500 pg of the cosmid 491, 2.5 units of TaKaRa ExTaq DNA polymerase was prepared. The reaction mixture was subjected to heat denaturation at 94°C for 3 minutes followed by a PCR of 30 cycles each consisting of 98°C for 10 seconds, 38°C for 10 seconds and 72°C for 40 seconds using Gene Amp PCR system 9600. Then, 2 µl each of the reaction mixtures was subjected to electrophoresis on agarose gel, and amplified DNA fragments were observed after staining with ethidium bromide.
   Single PCR-amplified fragments of varying sizes ranging from 400 bp to 6000 bp were obtained in 22 out of 92 reactions using the pool of primers I. The amplified fragments were subjected to removal of primers and salts from the reaction mixtures using Microcon-100, and direct sequencing using a sequencing primer having a nucleotide sequence of SEQ ID NO:2 (the tag sequence) according to a conventional method. As a result, a sequence of about 1746 nucleotides in the DNA fragment inserted into the cosmid 491 could be determined. Single PCR-amplified fragments of varying sizes ranging from 500 bp to 4000 bp were obtained in 20 out of 92 reactions using the pool of primers II. The amplified fragments were purified as described above and then subjected to direct sequencing. As a result, a sequence of 2045 nucleotides in the DNA fragment inserted into the cosmid 491 could be determined. Single PCR-amplified fragments of varying sizes ranging from 1100 bp to 4000 bp were obtained in 17 out of 77 reactions using the pool of primers IV. The amplified fragments were purified as described above and then subjected to direct sequencing using a sequencing primer 2 having a nucleotide sequence of SEQ ID NO:3. As a result, a sequence of 2614 nucleotides in the DNA fragment inserted into the cosmid 491 could be determined. Single PCR-amplified fragments of varying sizes ranging from 500 bp to 2900 bp were obtained in 23 out of 64 reactions using the pool of primers V. The amplified fragments were purified as described above and then subjected to direct sequencing using a sequencing primer 2 having a nucleotide sequence of SEQ ID NO:3. As a result, the nucleotide sequence of the DNA fragment inserted into the cosmid 491 could also be determined using the pool of primers V.
(3) Use of a commercially available ExTaq buffer in a PCR was also examined. The composition of the reaction mixture for a PCR was the same as that as described in (2) above except that a buffer for TaKaRa Ex-Taq DNA polymerase (Takara Shuzo) and 200 µM each of dNTPs were used. The reaction mixture was subjected to a PCR of 30 cycles each consisting of 98°C for 10 seconds, 38°C for 10 seconds and 72°C for 2 minutes using Gene Amp PCR system 9600. Then, 2 µl each of the reaction mixtures was subjected to electrophoresis on agarose gel, and amplified DNA fragments were observed after staining with ethidium bromide. As a result, results similar to those as described in (2) above were obtained for the respective pools of primers.

### Example 7

(1) A method for determining a nucleotide sequence of a genomic DNA from Pyrococcus furiosus was examined. A genomic DNA was prepared according to a conventional method.
(2) PCRs were carried out using the genomic DNA as a template, and a primer Pfu30F1 which has a nucleotide sequence of SEQ ID NO:9 and each one of the 24 primers (Nos. 49-72 in Table 1) among the 92 primers in the pool of primers I prepared in Example 1. 100 µl of a reaction mixture for a PCR containing 20 mM tris-acetate (pH 8.5), 50 mM potassium acetate, 3 mM magnesium acetate, 0.01% BSA, 300 µM each of dNTPs, 10 ng of the genomic DNA from Pyrococcus furiosus, 2.5 units of TaKaRa ExTaq DNA polymerase was prepared. The reaction mixture was subjected to heat denaturation at 94°C for 3 minutes followed by a PCR of 40 cycles each consisting of 98°C for 10 seconds, 50°C for 10 seconds and 72°C for 40 seconds using Gene Amp PCR system 9600. Then, 2 µl each of the reaction mixtures was subjected to electrophoresis on agarose gel, and amplified DNA fragments were observed after staining with ethidium bromide.
   Single PCR-amplified fragments of varying sizes ranging from 400 bp to 4000 bp were obtained in 8 out of 24 reactions using the pool of primers I. The amplified fragments were subjected to removal of primers and salts from the reaction mixtures using Microcon-100, and direct sequencing using a sequencing primer having a nucleotide sequence of SEQ ID NO:2 (the tag sequence) according to a conventional method. As a result, a sequence of about 1000 nucleotides in the genomic DNA could be determined, confirming the effectiveness of the method for determining a nucleotide sequence of a nucleic acid of the present invention.
(3) Use of a commercially available ExTaq buffer in a PCR was also examined. The composition of the reaction mixture for a PCR was the same as that as described in (2) above except that a buffer for TaKaRa Ex-Taq DNA polymerase (Takara Shuzo) and 200 µM each of dNTPs were used. The reaction mixture was subjected to heat denaturation at 94°C for 3 minutes followed by a PCR of 40 cycles each consisting of 98°C for 10 seconds, 50°C for 10 seconds and 72°C for 2 minutes using Gene Amp PCR system 9600. Then, 2 µl each of the reaction mixtures was subjected to electrophoresis on agarose gel, and amplified DNA fragments were observed after staining with ethidium bromide. As a result, results similar to those as described in (2) above were obtained for the pool of primers.

### Example 8

Cloning of Thermococcus litoralis RNase HII gene and Thermococcus celer RNase HII gene

### (1) Preparation of genomic DNAs

Cells of Thermococcus litoralis (purchased from Deutsche Sammlung von Mikroorganismen und Zellkulturen GmbH; DSM5473) or Thermococcus celer (purchased from Deutsche Sammlung von Mikroorganismen und Zellkulturen GmbH; DSM2476) were collected from 11 ml of a culture. The cells were independently suspended in 500 µl of 25% sucrose and 50 mM tris-HCl (pH 8.0). 100 µl of 0.5 M EDTA and 50 µl of 10 mg/ml lysozyme chloride (Nacalai Tesque) in water were added thereto. The mixture was reacted at 20°C for 1 hour. After reaction, 4 ml of a mixture containing 150 mM NaCl, 1 mM EDTA and 20 mM tris-HCl (pH 8.0), 50 µl of 20 mg/ml proteinase K (Takara Shuzo) and 250 µl of a 10% aqueous solution of sodium lauryl sulfate were added to the reaction mixture. The mixture was incubated at 37°C for 1 hour. After reaction, the mixture was subjected to phenolchloroform extraction and ethanol precipitation, air-dried and then dissolved in 100 µl of TE to obtain a genomic DNA solution.

### (2) Cloning of middle portions of RNase HII genes

Oligonucleotides RN-F1 (SEQ ID NO:10) and RN-R0 (SEQ ID NO:11) were synthesized on the basis of portions conserved among amino acid sequences of various thermostable RNase HIIs.

A PCR was carried out in a volume of 100 µl using 5 µl of the genomic DNA solution from Thermococcus litoralis or Thermococcus celer prepared in Example 8-(1) as a template, and 100 pmol each of RN-F1 and RN-R0 as primers. TaKaRa Taq (Takara Shuzo) was used as a DNA polymerase for the PCR according to the attached protocol. The PCR was carried out as follows: 50 cycles each consisting of 94°C for 30 seconds, 45°C for 30 seconds and 72°C for 1 minute. After reaction, Microcon-100 (Takara Shuzo) was used to remove primers from the reaction mixture and to concentrate the reaction mixture.

### (3) Cloning of upstream and downstream portions of RNase HII genes

The nucleotide sequences of the fragments of about 0.5 kb, TliF1R0 from Thermococcus litoralis and TceF1R0 from Thermococcus celer, obtained in Example 8-(2) were determined. A specific oligonucleotide TliRN-1 (SEQ ID NO:12) for cloning a portion upstream from TliF1R0 and a specific oligonucleotide TliRN-2 (SEQ ID NO:13) for cloning a portion downstream from TliF1R0 were synthesized on the basis of the determined nucleotide sequence. Furthermore, specific oligonucleotide TceRN-1 (SEQ ID NO:14) for cloning a portion upstream from TceF1R0 and a specific oligonucleotide TceRN-2 (SEQ ID NO:15) for cloning a portion downstream from TceF1R0 were synthesized on the basis of the determined nucleotide sequence. In addition, 48 primers as shown in Table 7 were synthesized. The tag sequence in Table 7 is shown in SEQ ID NO:16.

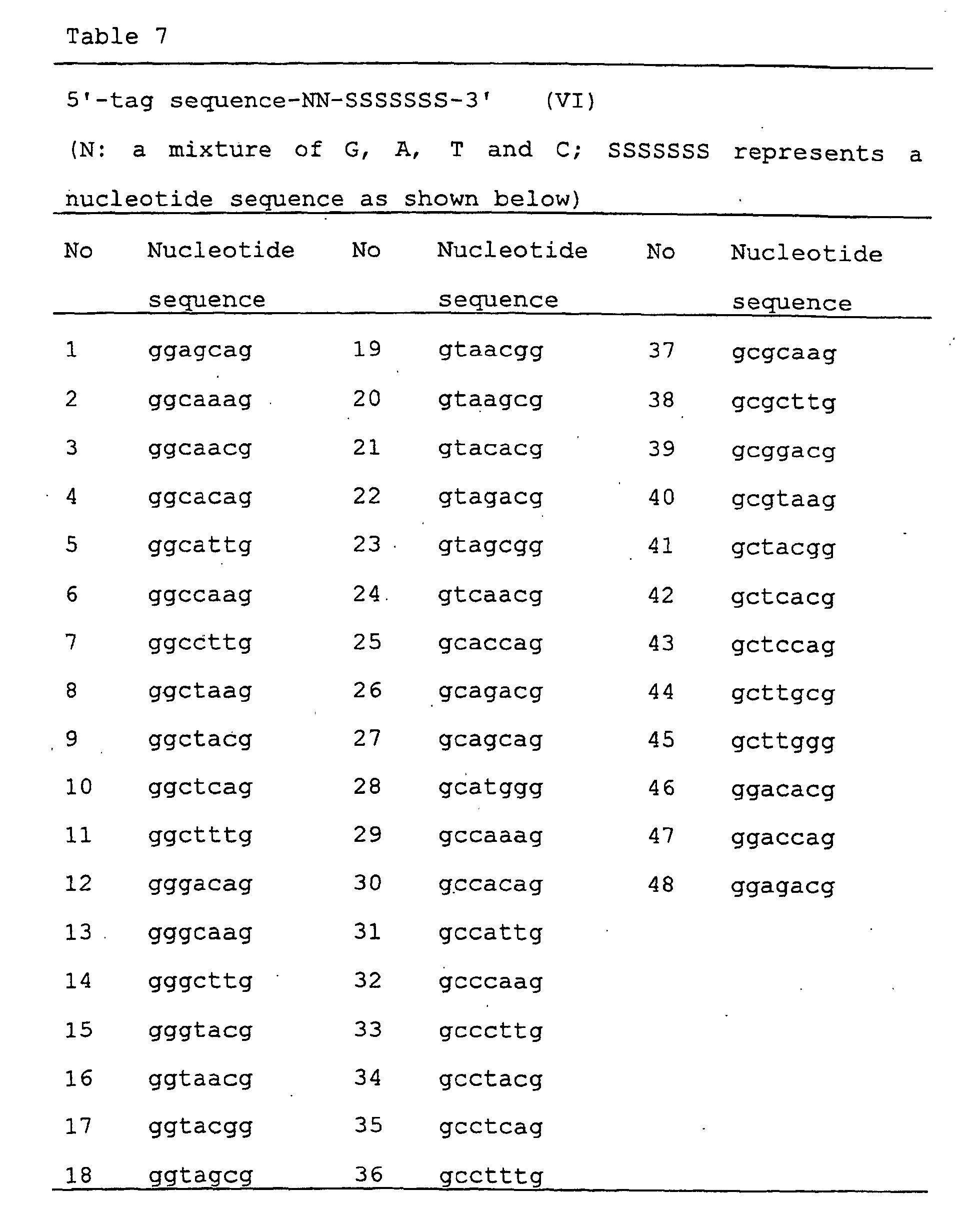

PCRs were carried out in reaction mixtures containing 1 µl of one of the genomic DNA solutions prepared in Example 8-(1) as a template, a combination of 20 pmol of TliRN-1 or 20 pmol of TliRN-2 and 20 pmol of each one of the 48 primers listed in Table 1, or a combination of 20 pmol of TceRN-1 or 20 pmol of TceRN-2 and 20 pmol of each one of the 48 primers listed in Table 1, 20 mM tris-acetate (pH 8.5), 50 mM potassium acetate, 3 mM magnesium acetate, 0.01% BSA, 30 µM each of dNTPs and 2.5 units of TaKaRa Ex Taq DNA polymerase (Takara Shuzo). PCRs were carried out as follows: incubation at 94°C for 3 minutes; and 40 cycles each consisting of 98°C for 10 seconds, 50°C for 10 seconds and 72°C for 40 seconds. A portion of each PCR product was subjected to electrophoresis on agarose gel. Microcon-100 (Takara Shuzo) was used to remove primers from reaction mixtures that resulted in single bands and to concentrate the reaction mixtures. The concentrates were subjected to direct sequencing to screen for fragments containing the upstream or downstream portions of the RNase HII. As a result, for Thermococcus litoralis, it was shown that an about 450-bp PCR-amplified fragment TliN7 contained the upstream portion of the RNase HII gene and an about 600-bp PCR-amplified fragment TliC25 and an about 400-bp PCR-amplified fragment TliC26 contained the downstream portion of the RNase HII gene, respectively. For Thermococcus celer, it was shown that an about 450-bp PCR-amplified fragment TceN24 contained the upstream portion of the RNase HII gene and an about 400-bp PCR-amplified fragment TceC29 contained the downstream portion of the RNase HII gene, respectively.

### (4) Cloning of entire RNase HII genes

The nucleotide sequence of a gene containing TliF1R0 as well as the upstream and downstream portions is shown in SEQ ID NO:17. The amino acid sequence of RNase HII deduced from the nucleotide sequence is shown in SEQ ID NO:18. Primers TliNde (SEQ ID NO:19) and TliBam (SEQ ID NO:20) were synthesized on the basis of the nucleotide sequence.

The nucleotide sequence of a gene containing TceF1R0 as well as the upstream and downstream portions is shown in SEQ ID NO:21. The amino acid sequence of RNase HII deduced from the nucleotide sequence is shown in SEQ ID NO:22. Primers TceNde (SEQ ID NO:23) and TceBam (SEQ ID NO:24) were synthesized on the basis of the nucleotide sequence.

A PCR was carried out in a volume of 100 µl using 1 µl of the Thermococcus litoralis genomic DNA solution obtained in Example 8-(1) as a template, and 20 pmol each of TliNde and TliBam as primers. Ex Taq DNA polymerase (Takara Shuzo) was used as a DNA polymerase for the PCR according to the attached protocol. The PCR was carried out as follows: 40 cycles each consisting of 94°C for 30 seconds, 55°C for 30 seconds and 72°C for 1 minute. An amplified DNA fragment of about 0.7 kb was digested with NdeI and BamHI (both from Takara Shuzo). Then, plasmids pTLI223Nd and pTLI204 were constructed by incorporating the resulting DNA fragment between NdeI and BamHI sites in a plasmid vector pTV119Nd (a plasmid in which the NcoI site in pTV119N is converted into a NdeI site) or pET3a (Novagen), respectively.

Furthermore, a PCR was carried out in a volume of 100 µl using 1 µl of the Thermococcus litoralis genomic DNA solution as a template, and 20 pmol each of TceNde and TceBam as primers. Pyrobest DNA polymerase (Takara Shuzo) was used as a DNA polymerase for the PCR according to the attached protocol. The PCR was carried out as follows: 40 cycles each consisting of 94°C for 30 seconds, 55°C for 30 seconds and 72°C for 1 minute. An amplified DNA fragment of about 0.7 kb was digested with NdeI and BamHI (both from Takara Shuzo). Then, plasmids pTCE265Nd and pTCE207 were constructed by incorporating the resulting DNA fragment between NdeI and BamHI sites in a plasmid vector pTV119Nd (a plasmid in which the NcoI site in pTV119N is converted into a NdeI site) or pET3a (Novagen), respectively.

### (5) Determination of nucleotide sequences of DNA fragments containing RNase HII genes

The nucleotide sequences of the DNA fragments inserted into pTLI223Nd, pTLI204, pTCE265Nd and pTCE207 obtained in Example 8-(2) were determined according to a dideoxy method.

Analyses of the determined nucleotide sequences revealed the existence of open reading frames presumably encoding RNase HIIs. The nucleotide sequence of the open reading frame in pTLI204 is shown in SEQ ID NO:25. The amino acid sequence of RNase HII deduced from the nucleotide sequence is shown in SEQ ID NO:26. "T" at position 484 in the nucleotide sequence of the open reading frame in pTLI204 was replaced by "C" in the nucleotide sequence of the open reading frame in pTLI223Nd. In the amino acid sequence, phenylalanine at position 162 was replaced by leucine.

The nucleotide sequence of the open reading frame in pTCE207 is shown in SEQ ID NO:27. The amino acid sequence of RNase HII deduced from the nucleotide sequence is shown in SEQ ID NO:28. "A" at position 14 in the nucleotide sequence of the open reading frame in pTCE207 was replaced by "G" in the nucleotide sequence of the open reading frame in pTCE265Nd. In addition, the nucleotides at positions 693 to 696 in the nucleotide sequence of the open reading frame in pTCE207 were missing in pTCE265Nd. In the amino acid sequence, glutamic acid at position 5 was replaced by glycine and phenylalanine at position 231 was missing.

### (6) Expression of RNase HII genes

Escherichia coli JM109 transformed with pTLI223Nd or pTCE265Nd was inoculated into 10 ml of LB medium containing 100 µg/ml of ampicillin and 1 mM IPTG and cultured with shaking at 37°C overnight. After cultivation, cells collected by centrifugation were suspended in 196 µl of Buffer A and sonicated. A supernatant obtained by centrifuging the sonicated suspension at 12,000 rpm for 10 minutes was heated at 70°C for 10 minutes and then centrifuged again at 12,000 rpm for 10 minutes to collect a supernatant as a heated supernatant. Similarly, Escherichia coli HMS174(DE3) transformed with pTLI204 or pTCE207 was inoculated into 10 ml of LB medium containing 100 µg/ml of ampicillin and cultured with shaking at 37°C overnight. After cultivation, cells collected by centrifugation were processed according to the procedure as described above to obtain a heated supernatant.

The enzymatic activities were measured for the heated supernatants. As a result, RNase H activities were observed for all transformants. Thus, the activity of the polypeptide was confirmed in spite of substitution in the nucleotide sequence or the amino acid sequence. As described above, it was demonstrated that a gene of interest can be conveniently and rapidly cloned according to the method of the present invention directly from a genome without constructing a library.

### Industrial Applicability

The present invention provides a rapid and low-cost method for determining a nucleotide sequence of a nucleic acid in which PCR products obtained by carrying out PCRs using a primer specific for a template and primers having defined nucleotide sequences are subjected to sequencing.

### Sequence Listing Free Text

SEQ ID NO:1: Artificially designed oligonucleotide.

SEQ ID NO:2: Artificially designed oligonucleotide.

SEQ ID NO:3: Artificially designed oligonucleotide.

SEQ ID NO:4: Artificially designed oligonucleotide.

SEQ ID NO:5: Artificially designed oligonucleotide.

SEQ ID NO:6: Artificially designed oligonucleotide.

SEQ ID NO:7: Artificially designed oligonucleotide.

SEQ ID NO:8: Artificially designed oligonucleotide.

SEQ ID NO:9: Artificially designed oligonucleotide.

SEQ ID NO:10: PCR primer RN-F1 for cloning a gene encoding a polypeptide having an RNaseHII activity from Thermococcus litoralis.

SEQ ID NO:11: PCR primer RN-R0 for cloning a gene encoding a polypeptide having a RNaseHII activity from Thermococcus litoralis.

SEQ ID NO:12: PCR primer TliRN-1 for cloning a gene encoding a polypeptide having a RNaseHII activity from Thermococcus litoralis.

SEQ ID NO:13: PCR primer TliRN-2 for cloning a gene encoding a polypeptide having a RNaseHII activity from Thermococcus litoralis.

SEQ ID NO:14: PCR primer TceRN-1 for cloning a gene encoding a polypeptide having a RNaseHII activity from Thermococcus celer.

SEQ ID NO:15: PCR primer TceRN-2 for cloning a gene encoding a polypeptide having a RNaseHII activity from Thermococcus celer.

SEQ ID NO:16: Designed oligonucleotid as tag sequence.

SEQ ID NO:19: PCR primer TliNde for amplifying a gene encoding a polypeptide having a RNaseHII activity from Thermococcus litoralis.

SEQ ID NO:20: PCR primer TliBam for amplifying a gene encoding a polypeptide having a RNaseHIII activity from Thermococcus litoralis.

SEQ ID NO:23: PCR primer TceNde for amplifying a gene encoding a polypeptide having a RNaseHII activity from Thermococcus celer.

SEQ ID NO:24: PCR primer TceBam for amplifying a gene encoding a polypeptide having a RNaseHIII activity from Thermococcus celer.

## Claims

1. A method for determining a nucleotide sequence of a nucleic acid, the method comprising:
(1) amplifying a template nucleic acid in the presence of each one of at least two primers each having a tag sequence, a primer specific for the template nucleic acid and a DNA polymerase, wherein the primer having a tag sequence has the tag sequence on the 5'-terminal side and a defined nucleotide sequence of three or more nucleotides on the 3'-terminal side; and
(2) subjecting a fragment amplified in step (1) above to direct sequencing.

2. The method according to claim 1, wherein the primer having a tag sequence has a structure represented by General Formula:
General Formula: 5'-tag sequence-Sₐ-3'
wherein "S" represents one nucleotide or a mixture of two or more nucleotides selected from the group consisting of G, A, T and C, and "a" represents an integer of three or more, provided that at least three S's in "Sₐ" represent one nucleotide selected from the group consisting of G, A, T and C.

3. The method according to claim 1, wherein the primer having a tag sequence is selected from the primers listed in Tables 1 to 5.

4. The method according claim 1, wherein the amplification of the template nucleic acid is carried out using a polymerase chain reaction (PCR).

5. The method according to claim 1, which further comprises selecting a pool of primers having a tag sequence that generates a substantially single-banded amplified fragment upon the amplification of the template nucleic acid.

6. The method according to claim 1, which further comprises purifying a substantially single-banded amplified fragment.

7. The method according to claim 4, wherein a pol I-type, α-type or non-pol I, non-α-type DNA polymerase, or a mixture of DNA polymerases is used in the PCR.

8. The method according to claim 1, wherein the DNA polymerase is selected from the group consisting of Taq DNA polymerase, Pfu DNA polymerase, Ex-Taq DNA polymerase, LA-Taq DNA polymerase, Z-Taq DNA polymerase, Tth DNA polymerase, KOD DNA polymerase and KOD dash DNA polymerase.

9. The method according to claim 1, which is carried out after preparing the template nucleic acid from a sample.

10. The method according to claim 9, wherein the template nucleic acid is provided in a form of a plasmid, phage, phagemid, cosmid, BAC or YAC library, or a genomic DNA or cDNA.

11. A pool of primers used for a method for determining a nucleotide sequence of a nucleic acid, which contains at least two primers each having a tag sequence, wherein the primer having a tag sequence has the tag sequence on the 5'-terminal side and a defined nucleotide sequence of three or more nucleotides on the 3'-terminal side, and wherein the method for determining a nucleotide sequence of a nucleic acid comprises:
(1) amplifying a template nucleic acid in the presence of each one of at least two primers each having a tag sequence, a primer specific for the template nucleic acid and a DNA polymerase, wherein the primer having a tag sequence has the tag sequence on the 5'-terminal side and a defined nucleotide sequence of three or more nucleotides on the 3'-terminal side; and
(2) subjecting a fragment amplified in step (1) above to direct sequencing.

12. The pool of primers according to claim 11, wherein the primer having a tag sequence has a structure represented by General Formula:
General Formula: 5'-tag sequence-Sₐ-3'
wherein "S" represents one nucleotide or a mixture of two or more nucleotides selected from the group consisting of G, A, T and C, and "a" represents an integer of three or more, provided that at least three S's in "Sₐ" represent one nucleotide selected from the group consisting of G, A, T and C.

13. The pool of primers according to claim 11, wherein the tag sequence in the primer having a tag sequence contains a sequence of a primer for sequencing.

14. A composition for determining a nucleotide sequence of a nucleic acid, which contains a pool of primers used for a method for determining a nucleotide sequence of a nucleic acid, wherein the pool of primers contains at least two primers each having a tag sequence,
wherein the primer having a tag sequence has the tag sequence on the 5'-terminal side and a defined nucleotide sequence of three or more nucleotides on the 3'-terminal side, and wherein the method for determining a nucleotide sequence of a nucleic acid comprises:
(1) amplifying a template nucleic acid in the presence of each one of at least two primers each having a tag sequence, a primer specific for the template nucleic acid and a DNA polymerase, wherein the primer having a tag sequence has the tag sequence on the 5'-terminal side and a defined nucleotide sequence of three or more nucleotides on the 3'-terminal side; and
(2) subjecting a fragment amplified in step (1) above to direct sequencing.

15. The composition according to claim 14, which further contains a DNA polymerase.

16. The composition according to claim 15, wherein the DNA polymerase is a pol I-type, α-type or non-pol I, non-α-type DNA polymerase, or a mixture of DNA polymerases.

17. The composition according to claim 16, wherein the DNA polymerase is selected from the group consisting of Taq DNA polymerase, Ex-Taq DNA polymerase, LA-Taq DNA polymerase, Z-Taq DNA polymerase, Tth DNA polymerase, KOD DNA polymerase and KOD dash DNA polymerase.

18. A kit used for a method for determining a nucleotide sequence of a nucleic acid, which contains a pool of primers, wherein the pool of primers contains at least two primers each having a tag sequence, wherein the primer having a tag sequence has the tag sequence on the 5'-terminal side and a defined nucleotide sequence of three or more nucleotides on the 3'-terminal side, and wherein the method for determining a nucleotide sequence of a nucleic acid comprises:
(1) amplifying a template nucleic acid in the presence of each one of at least two primers each having a tag sequence, a primer specific for the template nucleic acid and a DNA polymerase, wherein the primer having a tag sequence has the tag sequence on the 5'-terminal side and a defined nucleotide sequence of three or more nucleotides on the 3'-terminal side; and
(2) subjecting a fragment amplified in step (1) above to direct sequencing.

19. The kit according to claim 18, which further contains a DNA polymerase and a buffer for the DNA polymerase.

20. A kit used for a method for determining a nucleotide sequence of a nucleic acid, which is in a packed form and contains instructions that direct use of a pool of primers and a DNA polymerase, wherein the pool of primers contains at least two primers each having a tag sequence, wherein the primer having a tag sequence has the tag sequence on the 5'-terminal side and a defined nucleotide sequence of three or more nucleotides on the 3'-terminal side, and wherein the method for determining a nucleotide sequence of a nucleic acid comprises:
(1) amplifying a template nucleic acid in the presence of each one of at least two primers each having a tag sequence, a primer specific for the template nucleic acid and a DNA polymerase, wherein the primer having a tag sequence has the tag sequence on the 5'-terminal side and a defined nucleotide sequence of three or more nucleotides on the 3'-terminal side; and
(2) subjecting a fragment amplified in step (1) above to direct sequencing.

21. The kit according to claim 18 or 20, wherein the respective primers each having a tag sequence in the pool of primers are dispensed in predetermined positions.

22. A product consisting of a packing material and a reagent for determining a nucleotide sequence of a nucleic acid enclosed in the packing material, wherein the reagent contains a pool of primers and/or a DNA polymerase, and wherein description that the reagent can be used for determination of a nucleotide sequence is indicated in a label stuck to the packing material or instructions attached to the packing material.
